# EUROPEAN PATENT APPLICATION

(11) **EP 4 198 025 A1**
(43) Date of publication of application: **21.06.2023**
(21) Application number: 21214773.0
(22) Date of filing: 15.12.2021
(51) Int. Cl.: C07D 235/14, C07D 403/12, A61K 31/4184, A61P 31/04

(54) **BENZIMIDAZOLE DERIVATIVES FOR THE TREATMENT AND PROPHYLAXIS OF INFECTIOUS DISEASES**

(71) Applicant: Eberhard Karls Universität Tübingen Medizinische Fakultät, 72074 Tübingen (DE)
(72) Inventor: Schütz, Monika, 72070 Tübingen (DE); Renschler, Fabian, 72070 Tübingen (DE); Schweers, Jonas Malte, 3600 Thun (CH); Autenrieth, Ingo, 69121 Heidelberg (DE); Poso, Antti, 76150 Pieksämäki (FI); Kronenberger, Thales, 72076 Tübingen (DE); Kurki, Milla, 70210 Kuopio (FI); Turhanen, Petri, 70210 Kuopio (FI); Leini, Renne, 70210 Kuopio (FI); Schmidberger, Gregor, 72074 Tübingen (DE)
(74) Representative: Witte, Weller & Partner Patentanwälte mbB

(57) **Abstract**

The invention relates to a novel compound for the treatment and prophylaxis of a disease, especially a bacterial infectious disease, for reducing pathogen virulence and promoting host-induced clearance of infection and/or sensitizing bacteria to antibiotic treatment, a method for preparing said compound, a pharmaceutical composition comprising said compound, and to a method for the treatment and/or prophylaxis of a disease, preferably a bacterial infectious disease.

## Description

The invention relates to a novel compound for the treatment and prophylaxis of a disease, especially a bacterial infectious disease, for reducing pathogen virulence and promoting host-induced clearance of infection and/or sensitizing bacteria to antibiotic treatment, a method for preparing said compound, a pharmaceutical composition comprising said compound, and to a method for the treatment and/or prophylaxis of a disease, preferably a bacterial infectious disease.

### FIELD OF THE INVENTION

The invention relates to the field of pharmaceutically active compounds, more particular to the field of anti-infective compounds.

### BACKGROUND OF THE INVENTION

One major health problem of our days is the rising number of infections caused by antibiotic resistant pathogens. This narrows down the therapeutic options for treatment and in some cases makes antibiotic treatment even impossible. Cause of the emergence and spread of antibiotic resistance is the ample, and frequently also the inadequate use of antibiotics. Resistance development is forced by the high selection pressure caused by the administration of drugs that target essential processes (such as protein, RNA, DNA, cell wall or folic acid synthesis) in the pathogen. Additionally, as acting rather unspecific, antibiotic treatment has severe side effects. Those side effects are mostly caused by the largely unselective killing of bacteria inducing profound and long-lasting damage to the host's microbiome. As one nowadays knows, the microbiome plays an important role for multiple aspects of human health, therefore, the aim of innovative antibacterial therapeutics should be to limit their detrimental impact on the microbiota.

Urinary tract infections (UTI) are some of the most frequent bacterial infections, affecting ~ 150 million people per year worldwide. Approximately 1 % of all ambulatory visits in the U.S. account for patients presenting with urinary tract infections. It was estimated that the total costs of UTI sum up to 3.5 billion US-$ if health care costs and time missed from work are included. Uncomplicated UTI typically affects otherwise healthy individuals and causes morbidity especially in women of all ages. UTIs are caused by a number of Gram-negative and less commonly Gram-positive bacteria, but also fungi. The most frequent cause of uncomplicated UTI is uropathogenic *E. coli,* accounting for ~ 75 % of cases. Uncomplicated UTI is often treated by the prescription of antibiotics which may have severe side effects such as long-term alteration of vaginal and gut microbiota, candidiasis, and the development of resistance in bacterial organisms. If an uncomplicated UTI is observed at least 3 times within 12 months or 2 times within 6 months it is termed recurrent UTI. Recurrence may be caused by bacterial reinfection or by bacterial persistence, with reinfection being the more common mechanism. One measure to avoid recurrence is continuous low-dose antibiosis (6-12 months). This regimen is rather effective, but includes common side effects of antibiosis, i.e. vaginal or oral candidiasis, gastrointestinal symptoms due to disturbance of the endogenous microbiota and induces emergence of bacterial resistance.

Gastrointestinal infections account for a large burden of acute and chronic disease, with diarrhea being the most common manifestation. Bacteria such as *E. coli, S. flexneri, S. sonnei or Salmonella enterica* are important contributors to acute and chronic gastrointestinal infections. Symptomatic gastrointestinal infections are currently mostly treated by antibiotics, again associated with all typical side effects of antibiosis. In addition, antibiotic therapy may prolong bacterial shedding.

Against this background it is an object underlying the invention to provide a therapeutically and prophylactically usable compound which can be used to combat or counteract infections, especially bacterial infections, however which compound does not have the undesired side effects of traditional antibiotic compounds.

### SUMMARY OF THE INVENTION

This object is met by the provision of a compound of the formula 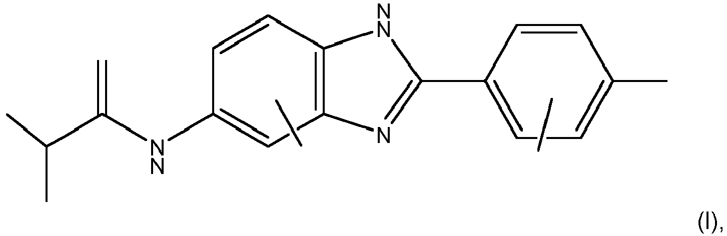 wherein R₁ and R₄ are independently selected from the group consisting of alkyl, aryl, heteroaryl, cycloalkyl with 3-12 C-atoms, and heterocycloalkyl with 3-12 C-atoms; R₂ is selected from the group consisting of alkyl, aryl, and heteroaryl; and R₃ is a functional group having a molecular weight of 200 g/mol or less; and the salts thereof, the solvates thereof and the solvates of the salts thereof.

The invention furthermore provides a method for preparing a compound of the formula

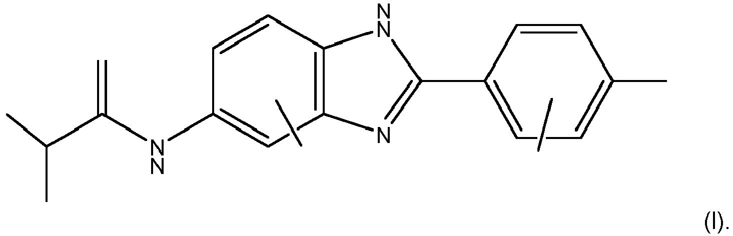

The method comprises: reacting a compound of the formula 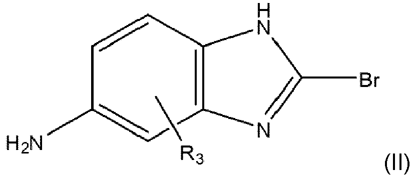 with a compound of the formula 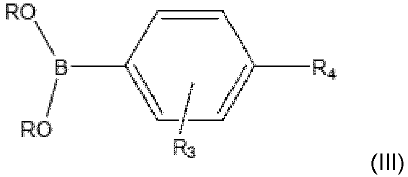 to obtain a compound of the formula 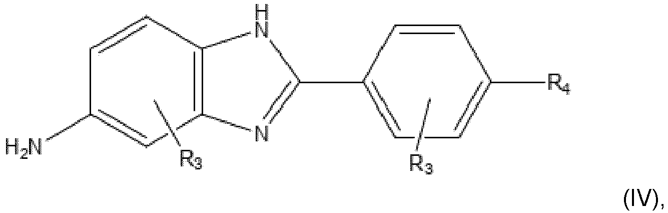 and reacting the compound of the formula (IV) with (CHR₁R₂)COR" to obtain the compound of the formula (I), wherein R₁ and R₄ are independently selected from the group consisting of alkyl, aryl, heteroaryl, cycloalkyl with 3-12 C-atoms, and heterocycloalkyl with 3-12 C-atoms; R₂ is selected from the group consisting of alkyl, aryl, and hetero aryl; R₃ is independent from each other a functional group having a molecular weight of 200 g/mol or less; and R" is selected from the group consisting of OH, OOH, CI, Br, OR, OOCOR‴, wherein R‴ is selected from the group consisting of alkyl with 3-8 C-atoms, cycloalkyl with 3-8 C-atoms, and aryl.

It is preferred that (CHR₁R₂)COR" is (CHR₁R₂)COOH.

It is still preferred that the method encompasses purifying the compound of the formula (IV) and/or purifying the compound of the formula (VI). It is still preferred that the compound of the formula (VI) is in form of a salt thereof, a solvate thereof and a solvate of the salt thereof.

The term "alkyl" as used herein refers to a saturated or unsaturated straight or branched chain hydrocarbon group of 1-40 C-atoms, preferably of 1-10 C-atoms, such as 2-9 C-atoms, 3-8 C-atoms, 4-7 C-atoms, and 5-6 C-atoms. Optionally, an alkyl group can be substituted, for example, with one or more, such as 1-4 groups, preferably 2 -3 groups, independently selected from, for example, F, Cl, Br, I, alkyl, alkenyl, alkynyl, OCF₃, NO₂, CN, NC, OH, SH, alkoxy, thioalkoxy, amino, CO₂H, CO₂, C(O)NH₂, oxo (=O), alkyl, aryl, and heteroaryl. Examples of alkyl groups include methyl, ethyl, n-propyl, isopropyl, n-butyl, isobutyl, tert-butyl, n-hexyl, etc.

"Heteroalkyl" is defined as alkyl, except the heteroalkyl contains at least one heteroatom, preferably two or three heteroatoms, independently selected from the group consisting of oxygen, nitrogen, and sulfur, preferably nitrogen.

The term "aryl" as used herein refers to a monocyclic or polycyclic aromatic group, preferably a bicyclic aromatic group, such as phenyl or naphthyl. Aryl groups optionally can be substituted, for example, with one or more, such as 1-4 groups, preferably 2 -3 groups, independently selected from, for example, F, Cl, Br, I, alkyl, alkenyl, alkynyl, alkylene, OCF₃, NO₂, CN, NC, OH, SH, alkoxy, thioalkoxy, amino, CO₂H, CO₂, alkyl, aryl, and heteroaryl. Exemplary aryl groups include phenyl, naphthyl, tetrahydronaphthyl, chlorophenyl, methylphenyl, methoxyphenyl, trifluoromethylphenyl, nitrophenyl, 2,4-methoxychlorophenyl, etc.

As used herein, the term "heteroaryl" refers to a monocyclic or polycyclic, such as bicyclic, ring system containing one or two aromatic rings and containing at least one nitrogen, oxygen, or sulfur atom in an aromatic ring. Heteroaryl groups optionally can be substituted, for example, with one or more, such as 1-4 groups, preferably 2 -3 groups, independently selected from, for example, F, Cl, Br, I, alkyl, alkenyl, alkynyl, alkylene, OCF₃, NO₂, CN, NC, OH, SH, alkoxy, thioalkoxy, amino, CO₂H, CO₂, alkyl, aryl, and heteroaryl. Examples of heteroaryl groups include, but are not limited to, thienyl, furyl, pyridyl, oxazolyl, quinolyl, thiophenyl, isoquinolyl, indolyl, triazinyl, triazolyl, isothiazolyl, isoxazolyl, imidazolyl, benzothiazolyl, pyrazinyl, pyrimidinyl, thiazolyl, and thiadiazolyl. Further examples of heteroaryl include indene having 1-3 C-atoms substituted with N, and naphthalene having 1-3 C-atoms substituted with N.

The term "cycloalkyl" as used herein refers to a cyclic hydrocarbon group, e.g., cyclopropyl, cyclobutyl, cyclohexyl, and cyclopentyl. The cyclic hydrocarbon group may be saturated or unsaturated. The cycloalkyl groups optionally can be substituted, for example, with one or more, such as 1-4 groups, preferably 2 -3 groups, independently selected from, for example, F, Cl, Br, I, alkyl, alkenyl, alkynyl, alkylene, OCF₃, NO₂, CN, NC, OH, SH, alkoxy, thioalkoxy, amino, CO₂H, CO₂, alkyl, aryl, and heteroaryl. The cycloalkyl has 3-12 C-atoms, such as 4-11 C-atoms, 5-10 C-atoms, 6-9 C-atoms, and 7-8 C-atoms.

The "heterocycloalkyl" is defined as cycloalkyl, except the ring contains one to three heteroatoms independently selected from the group consisting of oxygen, nitrogen, and sulfur. The heterocycloalkyl groups can be saturated or partially unsaturated ring systems optionally substituted with, for example, one to three groups, independently selected from the group consisting of alkyl, alkylene, OH, C(O)NH₂, NH₂, oxo (=O), aryl, haloalkyl, halo, and OH. Examples of heterocycloalkyl groups include piperidine, tetrahydrofuran, tetrahydropyran, dihydrofuran, morpholine, thiophene, dicyclopentadiene having 1-3 C-atoms substituted with N, etc.

Heterocycloalkyl groups optionally can be further N-substituted with alkyl, hydroxyalkyl, alkylene aryl, or alkylene heteroaryl. The heterocycloalkyl has 3-12 C-atoms, such as 4-11 C-atoms, 5-10 C-atoms, 6-9 C-atoms, and 7-8 C-atoms. Preferably, the heterocycloalkyl group is a monocyclic or bicyclic ring, more preferably, a monocyclic ring, wherein the ring comprises from 2 to 6 C-atoms and from 1 to 3 heteroatoms. More preferably the molecular weight of a heterocycloalkyl residue has a molecular weight of ≤ 250 g/mol, such as ≤ 200 g/mol.

The term "functional group having a molecular weight of 200 g/mol or less" refers to any functional group, such as F, Cl, Br, I, alkyl, alkenyl, alkynyl, OCF₃, NO₂, CN, NC, OH, SH, alkoxy, thioalkoxy, amino, CO₂H, CO₂, alkyl, aryl, and heteroaryl. The functional group preferably has a molecular weight of 200 g/mol or less, still preferably 150 g/mol or less, still preferably 100 g/mol or less, such as in a range of from 10 g/mol to 90 g/mol, 20 g/mol to 80 g/mol, 30 g/mol to 70 g/mol, 40g/mol to 60 g/mol, and 50 g/mol to 55 g/mol.

The term "alkenyl" used herein refers to a straight or branched chain hydrocarbon group of 2-10 C-atoms, preferably 3-6 C-atoms, or 4-5 C-atoms, containing at least one carbon double bond including, e.g., 1-propenyl, 2-propenyl, 2-methyl-1-propenyl, 1-butenyl, 2-butenyl. The term "cycloalkenyl" refers to a cycloalkyl group having one or more double bonds.

"Heterocycloalkenyl" refers to a cycloalkenyl group having one or more heteroatoms (e.g., N, S, O, or combinations thereof).

The term "alkynyl" used herein refers to a straight or branched chain hydrocarbon group of 2-10 C-atoms containing at least one carbon triple bond, such as 1-propynyl, 2-propynyl, 1-butynyl, and 2-butynyl.

The term "alkylene" used herein refers to an alkyl group having a substituent. For example, the term "alkylene aryl" refers to an alkyl group substituted with an aryl group.

The molecular sizes of the residues, including alkyl, aryl, heteroaryl, cycloalkyl with 3-12 C-atoms, heterocycloalkyl with 3-12 C-atoms, and a functional group having a molecular weight of 200 g/mol or less, is in a range ensuring that the present compound do not exhibit a sterical hindrance that negatively affects or even impedes the biological activity. Therefore, the residues R₁, R₂ and R₄ may each exhibit an overall molecular weight of 500 g/mol or less, such as 400 g/mol or less, 300 g/mol or less, or 250 g/mol or less.

The present compound may, depending on its specific structure, exist in stereoisomeric forms (enantiomers, diastereomers). The invention therefore also encompasses the enantiomers or diastereomers and respective mixtures thereof. The stereoisomerically uniform constituents can be isolated in a known manner from such mixtures of enantiomers and/or diastereomers. If the compound of the invention may occur in tautomeric forms, the present invention encompasses all tautomeric forms.

Salts preferred for the purposes of the present invention are physiologically acceptable salts of the compound of the invention. Also encompassed, however, are salts which are themselves not suitable for pharmaceutical applications but can be used for example for the isolation or purification of the compound of the invention.

Examples of pharmaceutically acceptable salts of the compound of formula (I) include salts of inorganic bases like ammonium salts, alkali metal salts, in particular sodium or potassium salts, alkaline earth metal salts, in particular magnesium or calcium salts; salts of organic bases, in particular salts derived from cyclohexylamine, benzylamine, octylamine, ethanolamine, diethanolamine, diethylamine, triethylamine, ethylenediamine, procaine, morpholine, pyrroline, piperidine, N-ethylpiperidine, N-methylmorpholine, piperazine as the organic base; or salts with basic amino acids, in particular lysine, arginine, ornithine and histidine.

Examples of pharmaceutically acceptable salts of the compound of formula (I) also include salts of inorganic acids like hydrochlorides, hydrobromides, sulfates, phosphates or phosphonates; salts of organic acids, in particular acetates, formates, propionates, lactates, citrates, fumarates, maleates, benzoates, tartrates, malates, methanesulfonates, ethanesulfonates, toluenesulfonates or benzenesulfonates; or salts with acidic amino acids, in particular aspartate or glutamate.

Solvates for the purposes of the invention refer to those forms of the compound of the invention, which in the solid or liquid state form a complex by coordination with solvent molecules. Hydrates are a specific form of solvates in which the coordination takes place with water.

The compound of the invention may also be complexed, e.g. with iron, calcium, etc., in which the compound may act as a ligand, so that a corresponding complex is also subject of the present invention.

According to a preferred embodiment, at least one of R₁, R₂, R₃, and R₄ is as follows: R₁ is selected from the group consisting of an indene having 1-3 C substituted with N, a naphthalene having 1-3 C substituted with N, dicyclopentadiene having 1-3 C substituted with N, biphenyl, naphthalene, and anthracene; R₂ is selected from the group consisting of H, OR, SR, NHR, NRR', wherein R and R' are independently selected from the group consisting of alkyl with 1-8 C-atoms, cycloalkyl with 3-8 atoms, and aryl; R₃ is selected from the group consisting of H, OH, NH₂, SH, COOH, F, Cl, Br, and I; and R₄ is selected from the group consisting of NH₂, NHR, NRR', and cycloalkyl with 3-12 C-atoms, wherein R and R' are independently selected from the group consisting of alkyl with 1-8 C-atoms, cycloalkyl with 3-8 C-atoms, and aryl. It is preferred that R₁ and R₂ are as indicated above. Still preferably R₁ and R₃, R₁ and R₄, R₂ and R₃, R₂ and R₄, or R₃ and R₄ are as indicated above. More preferably, R_{1,} R₂, and R₃; R_{1,} R₂, and R₄; R₂, R₃, and R₄ are as indicated above. Even more preferably, R₁ to R₄ are as indicated above.

It is preferred that R₁ is selected from the group consisting of 2,3-dihydro-1 H-indene, indene, indoline, 3H-indole, 1H-indole, 2H-isoindole, indolizine, 1H-indazole, 2H-indazole, benzimidazole, 7-azaindole, 4-azaindole, 5-azaindole, and 6-azaindole, biphenyl, naphthalene, and anthracene. Optionally, these residues have one or more substituents selected from the group consisting of OH, NH₂, SH, COOH, F, Cl, Br, and I. More preferably, these residues are unsubstituted. It is still preferred that R₂ is selected from the group consisting of H, and unsubstituted alkyl with 1-5 C-atoms, more preferably H, methyl, and ethyl. Optionally, these residues have one or more substituents selected from the group consisting of OH, NH₂, SH, COOH, F, Cl, Br, and I. More preferably, these residues are unsubstituted. It is still preferred that R₃ is selected from the group consisting of H, OH, NH₂, SH, COOH, F, Cl, Br, and I. It is still preferred that R₄ is selected from the group consisting of NH₂, NHR, NRR', wherein R and R' are independently selected from the group consisting of alkyl, more preferably methyl or ethyl, cycloalkyl with 3-8 C-atoms, aryl, and heteroaryl, more preferably piperazinyl. Optionally, these residues have one or more substituents selected from the group consisting of OH, NH₂, SH, COOH, F, Cl, Br, and I. More preferably, these residues are unsubstituted. It is preferred that R₁ and R₂ are as indicated above. Still preferably R₁ and R₃, R₁ and R₄, R₂ and R₃, R₂ and R₄, or R₃ and R₄ are as indicated above. More preferably, R_{1,} R₂, and R₃; R_{1,} R₂, and R₄; R₂, R₃, and R₄ are as indicated above. Even more preferably, R₁ to R₄ are as indicated above.

More preferably, R₁ is selected from the group consisting of 2,3-dihydro-1 H-indene, indene, indoline, 3H-indole, 1H-indole, 2H-isoindole, indolizine, 1H-indazole, 2H-indazole, benzimidazole, 7-azaindole, 4-azaindole, 5-azaindole, and 6-azaindole. Still more preferably, R₂ is selected from the group consisting of H, methyl, and ethyl, even more preferably R₂ is H. Still more preferably R₃ is selected from the group consisting of H, F, Cl, Br, and I, even more preferably R₃ is H. It is still preferred that R₄ is selected from the group consisting of NH₂, NHR, NRR', wherein R and R' are independently selected from the group consisting of methyl, ethyl, and piperazinyl, preferably methyl and ethyl. It is preferred that R₁ and R₂ are as indicated above. Still preferably R₁ and R₃, R₁ and R₄, R₂ and R₃, R₂ and R₄, or R₃ and R₄ are as indicated above. More preferably, R_{1,} R₂, and R₃; R_{1,} R₂, and R₄; R₂, R₃, and R₄ are as indicated above. Even more preferably, R₁ to R₄ are as indicated above.

The present compounds may by synthesized according to the reaction scheme 1 depicting a two-step synthesis, in which the residues R₁ to R₄ are as indicated above. A first reaction step involves the Suzuki-Miyaura reaction, also known as Suzuki rection or Suzuki coupling. A second step involves a conventional amidation. The amidation is shown with the compound (CHR₁R₂)COOH. It will be appreciated that instead of (CHR₁R₂)COOH, the carboxylic acid may be activated, i.e. the compound (CHR₁R₂)COR".

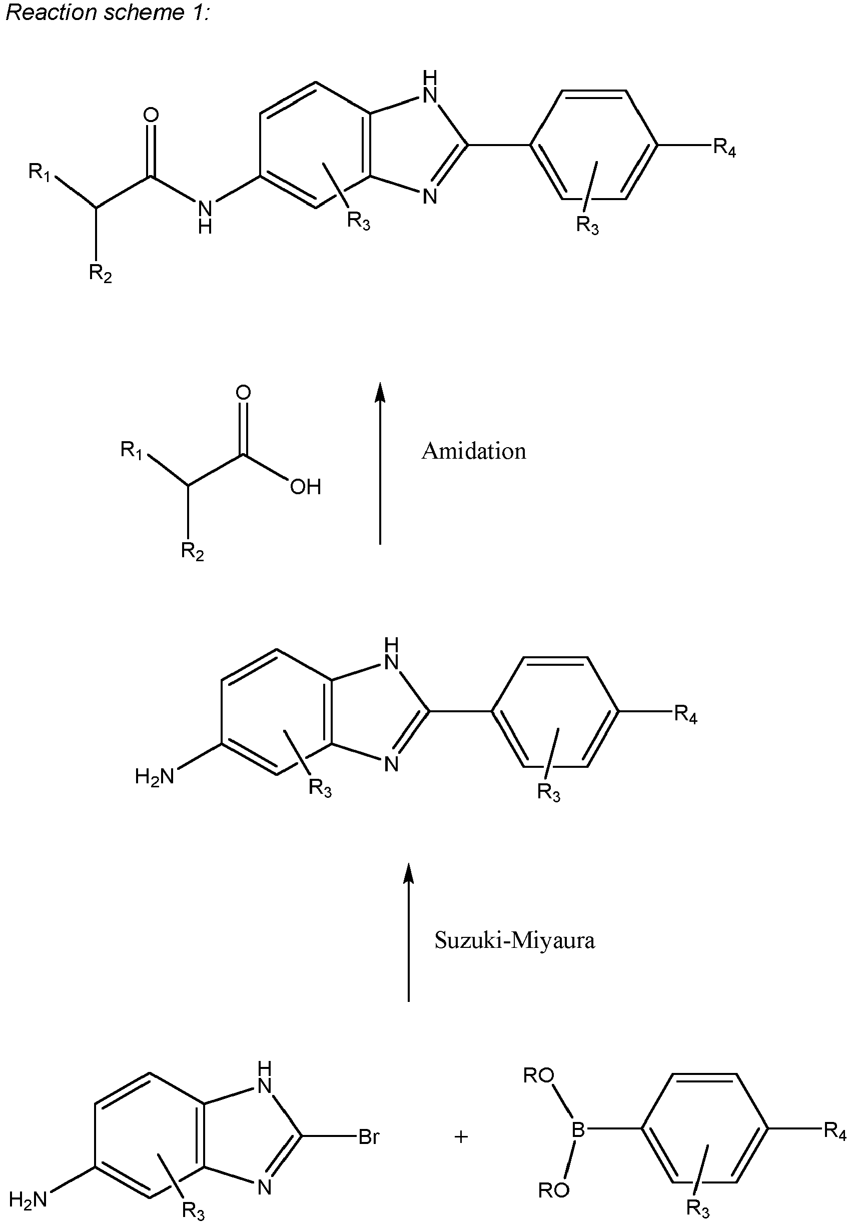

It is well known in the art that the Suzuki-Miyaura reaction is based on the Pd-catalysed coupling of an aryl- or vinyl halogenide with an aryl- or vinyl- boronic acid.

For instance, the Suzuki-Miyaura reaction may be carried out by dissolving 1 equivalent of the compound of the formula (II) and about 1.01 to 1.25 equivalents, such as 1.05 to 1.15 eq., or 1.08 to 1.11 eq., or 1.09 to 1.10 eq., of the compound of the formula (III), and a base, preferably a tertiary amine, such as triethylamine, preferably about 10 to 15 eq., such as 12.2 eq., in a mixture of an organic solvent, such as 1,4-dioxane, and H₂O (preferably in a relationship of about 3-5 : 1, such as 3.5 - 4.5: 1, or 4 : 1). About 0.2 to 0.5 eq., such as 0.25 to 0.4 eq., 0.3 to 0.35 eq. or 0.33 to 0.34 eq., of a palladium compound, preferably Pd(dppf)Cl₂, are added to the reaction mixture and stirred for an elongated period, such as 12 to 60 hours, such as 24 to 48 h, or 40 h, at a temperature affording reflux of the solvent, such as about 95°C or more, such as 100 to 110°C. The reaction mixture may by purified according to the skilled persons knowledge, e.g. by filtering, such as filtering through a solid filter, e.g., a celite material.

The amidation may be carried out by dissolving the compound of the formula (IV) and (CHR₁R₂)COR" in an amount of 0.7 to 1.3 eq. compound of the formula (IV) and 1 eq. of (CHR₁R₂)COR", preferably 1.2 eq. compound of the formula (IV) and 1 eq. of (CHR₁R₂)COR", in an organic solvent, such as acetonitrile. Approx. 1.0 to 1.4 eq., preferably 1.2 eq., of a carbodiimide, such as 1-Ethyl-3-(3-dimethylaminopropyl)carbodiimidehydrochloride (EDC-HCI), and approx. 1.0 to 1.4 eq., preferably 1.2 eq., of an alcohol hydrate, such as HOBt hydrate, are added. The reaction mixture may be stirred for, e.g., 24 to 48 hours, such as 40 h, at a temperature of, e.g., about 30 to 50°C, such as 40°C. The reaction mixture may by purified according to the skilled persons knowledge, e.g. by subjecting the reaction mixture to High Performance Counter Current Chromatography or High Performance Liquid Chromatography.

It will be appreciated that other synthesis methods may be used as well.

In addition, the synthesis may require the use of a protecting group. In general, a "protecting group" or a "protective group" is a reversibly formed derivative of an existing functional group in a molecule. The protective group is temporarily attached to decrease reactivity so that the protected functional group does not react under synthetic conditions to which the molecule is subjected in one or more subsequent steps. For instance, protecting an amine as a carbamate enables other functional groups to undergo selective reactions with electrophiles whereby the carbamate (protected amino group) is left intact. This involves two additional synthetic steps: the step to form the protected intermediate and a deprotection once the additional selective synthetic steps have been completed. The skilled person is well aware about suitable protecting groups as well as the techniques involved in protecting and deprotecting a given compound.

In another embodiment of the invention the compound of the formula (I) is N-(2-(4-(dimethylamino)phenyl)-1H-benzo[d]imidazol-5-yl)-2-(1H-indazol-1-yl)acetamide, preferably having the following formula

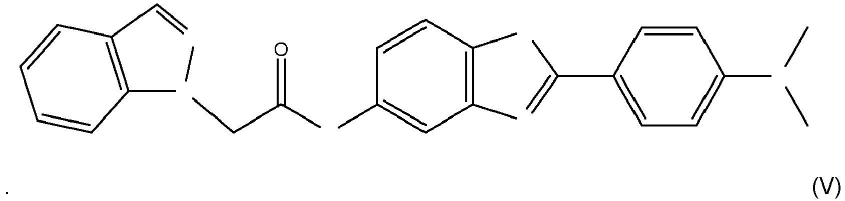

In still another embodiment of the invention the compound of the formula (I) is N-(2-(4-(dimethylamino)phenyl)-1H-benzo[d]imidazol-5-yl)-2-(2H-indazol-2-yl)acetamide, preferably having the following formula

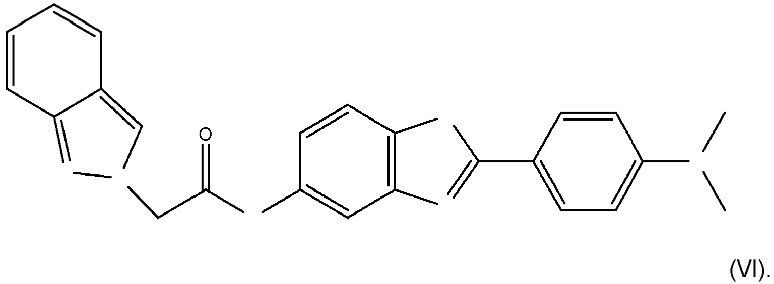

This compound, out of a range of thousands tested by the inventors, showed best overall performance as, e.g., demonstrated in well-established tests such as primary assay, MST measurements, nanoDSF measurements, docking studies, 2D-NMR titration experiments, etc. The compound showed a very high SurA inhibition already at low concentrations and affected the thermal stability of SurA. Additional modelling studies suggest a stable interaction of this preferred compound in the interface of the NC-core and P1 domains. Details can be found in the experiments further below (the compound of the formula VI is there referred to as compound QHL-24).

The inventors have found that the compound according to the invention does not necessarily kill the pathogens, but essentially disarms them, i.e. render them less virulent, and enables the host to clear the infection, and at the same time largely preserves the endogenous microbiota, poses an excellent alternative and additional strategy to resolve bacterial infections.

Against this background the invention further relates to the compound according to the invention for use in the treatment and/or prophylaxis of a disease, preferably an infectious disease, further preferably a bacterial disease, further preferably an infection by a Gram-negative bacterium, highly preferably a bacterium selected from the group consisting of: *Escherichia coli, Pseudomonas ssp., Klebsiella spp., Yersinia spp., Shigella spp., Serratia spp., Morganella spp., Enterobacter spp.*

The inventors have realized that the compound according to the invention is capable to inhibit the activity of SurA. SurA is a periplasmic chaperone which is essential for shuttling outer membrane (OM) proteins across the periplasm of bacteria, especially Gram-negative bacteria, and thereby shapes the composition and barrier function of the OM. Other than antibiotics the compound according to the invention inhibits a non-essential factor that is crucial for the biogenesis of several proteins important for virulence.

By inhibition of SurA the compound according to the invention renders the bacteria less virulent and more susceptible to killing by the host immune system, e.g. by reducing the exposure of adhesins mediating attachment to host cells, of flagella that enable the bacteria to move, and of cell surface factors negatively regulating the attack of the bacteria by the host complement system.

As SurA is located in the periplasm, the compound according to the invention only has to traverse the OM to get to its target as compared to cytoplasmic targets which are additionally shielded by the inner membrane. In contrast to manipulating single porins, efflux pumps or proteins involved in LPS biogenesis, the compound according to the invention attacks a target whose inhibition will induce a more profound rearrangement of the bacterial OM. By the inhibition of SurA the compound will interfere with the biogenesis of several factors known to be decisive for virulence, e.g. iron uptake receptors. Amongst the factors strikingly influenced by a knockout of SurA is LptD, which is already identified as a promising target in pathogenic bacteria.

SurA is highly conserved throughout bacteria, especially Gram-negative bacteria. This allows the targeting of a wide range of different potentially pathogenic bacteria by the compound of the invention. On the other side, however, the inventors have found that the compound according to the invention acts very specific on SurA. Thus, by the inhibition of SurA the host microbiome composition will only be changed to a minor degree, if at all. In contrast, antibiotic treatment causes profound and often long-lasting disturbance of the microbiota. The treatment of bacterial infections with the compound according to the invention is therefore significantly more advantageous and has fewer side effects than that with antibiotic substances.

The inventors have found that such infections are particularly well treated or prevented with the compound according to the invention, which are caused by the bacteria mentioned, which are also of particular clinical relevance.

In an embodiment of the invention said infectious disease is caused by a multi-drug resistant (MDR) bacterium, preferably an MDR Gram-negative bacterium, preferably an ESKAPE bacterium, including *Klebsiella pneumoniae, Acinetobacter baumannii, Pseudomonas aeruginosa,* and *Enterobacter spp.*

The inventors realized that the compound according to the invention results in an overall destabilization of the bacterial outer membrane. This destabilization enhances the entry of antibiotics into the cells. This effect can re-sensitize even multiresistant bacteria to antibiotic treatment, even bacteria belonging to the ESKAPE group.

In another embodiment of the invention said infectious disease is caused by a SurA-expressing bacterium.

Through this measure, the invention makes use of the mechanism of action on which the compound found is based in an advantageous manner.

In another embodiment of the invention the compound is configured for the treatment and/or prophylaxis of urinary tract infections (UTI) and/or gastrointestinal infections.

This measure has the advantage that an alternative to the current antibiotic treatment of UTI is provided by means of which common side effects of antibiosis, e.g. vaginal or oral candidiasis, gastrointestinal symptoms due to disturbance of the endogenous microbiota, induction of bacterial resistance, etc., are avoided.

According to the invention, UTI also includes uncomplicated UTI and recurrent uncomplicated UTI.

In another embodiment of the invention the compound according to the invention is configured for the treatment and/or prophylaxis of blood stream infections, preferably to re-sensitize an MDR bacterium for antibiotics.

The inventors found that the compound according to the invention causes defects in the biogenesis of the bacterial outer membrane proteins. According to the inventors, this damage of the bacterial membrane leads to a significant increase in the susceptibility and accessibility of the treated bacteria to antibiotic agents. As a result, even multiresistant bacterial strains can again be treated with antibiotics.

In an embodiment of the invention the compound is configured for a use as a SurA inhibitor.

According to the invention such use includes both a use *in vivo,* i.e. in a living being, such as a mammal or human, but also *in vitro,* e.g. in biological cells, tissues or organs, respectively.

Another subject-matter of the invention relates to a pharmaceutical composition comprising the compound according to the invention and a pharmaceutically acceptable carrier.

"Pharmaceutically acceptably carriers" are well known to the skilled person. They allow a proper formulation of the compound according to the invention and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminium hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The features, characteristics, embodiments and advantages explained for the compound and method according to the invention apply *mutatis mutandis* to the pharmaceutical composition of the invention.

In an embodiment of the pharmaceutical composition according to the invention the latter additionally comprises an antibiotic compound.

This measure makes use of the property of the compound according to the invention to make potentially pathogenic bacteria increasingly or again accessible to treatment with antibiotic substances. The combination of the compound according to the invention and an antibiotic substance consequently leads not only to an additive but also to a synergistic effect.

Another subject-matter of the invention relates to a method for the treatment and/or prophylaxis of a disease, preferably an infectious disease, of a living being, comprising the administration of the compound and/or the pharmaceutical composition according to the invention, to a living being in need thereof.

The features, characteristics, embodiments and advantages explained for the compound and pharmaceutical composition according to the invention apply *mutatis mutandis* to the afore-mentioned method of the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

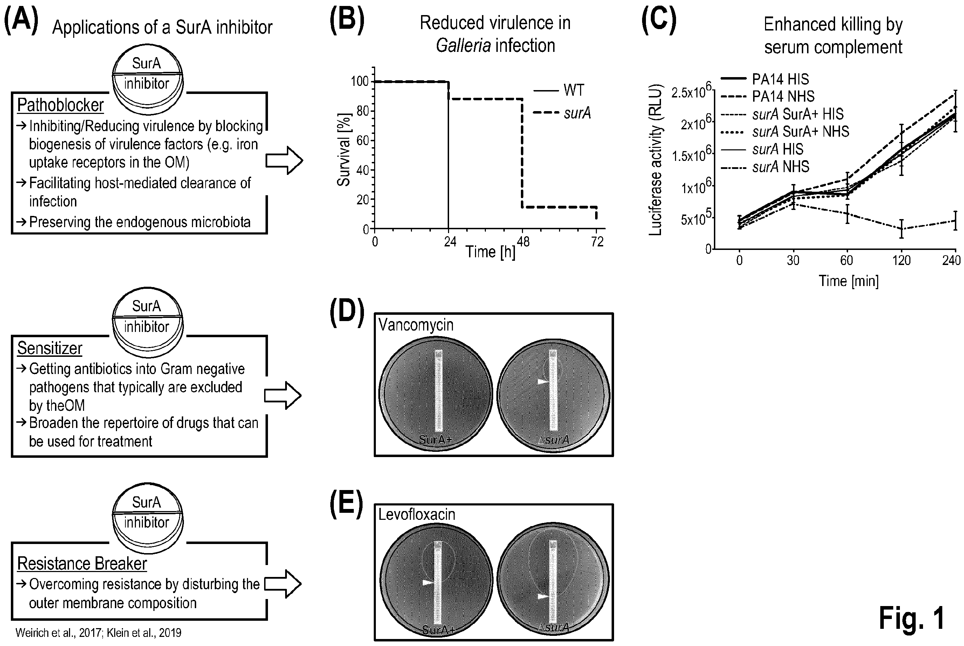

- Fig. 1:: Potential applications of a SurA inhibitor. (A) The versatile applications of a SurA inhibitor and its presumed effects on bacterial virulence and antibiotic resistance. (B) Depletion of SurA reduces virulence of e.g. *P. aeruginosa* in a *Galleria melonella* infection model. Please note that depletion of SurA expression is halted once bacteria are introduced into the larvae, thus the inventors expect much better survival if SurA was depleted throughout the entire experiment. (C) Depletion of SurA renders Pa highly sensitive to killing by the complement system in normal human serum (NHS). As a control, bacteria were incubated in heat-inactivated human serum (HIS). Indicated bacterial strains were grown for various time periods in NHS or HIS and subsequently luciferase activity (which is directly proportional to the ATP levels of viable cells in a sample) was measured. Data depict the mean and SD of luciferase activity measured of 3 independent experiments performed in triplicates. (D) E-test indicating that the lack of SurA impairs OM integrity and thereby renders Pa susceptible to vancomycin. (E) E-test demonstrating that the lack of SurA significantly reduces the MICs for antibiotics, e.g. Levofloxacin, and can even break resistance.

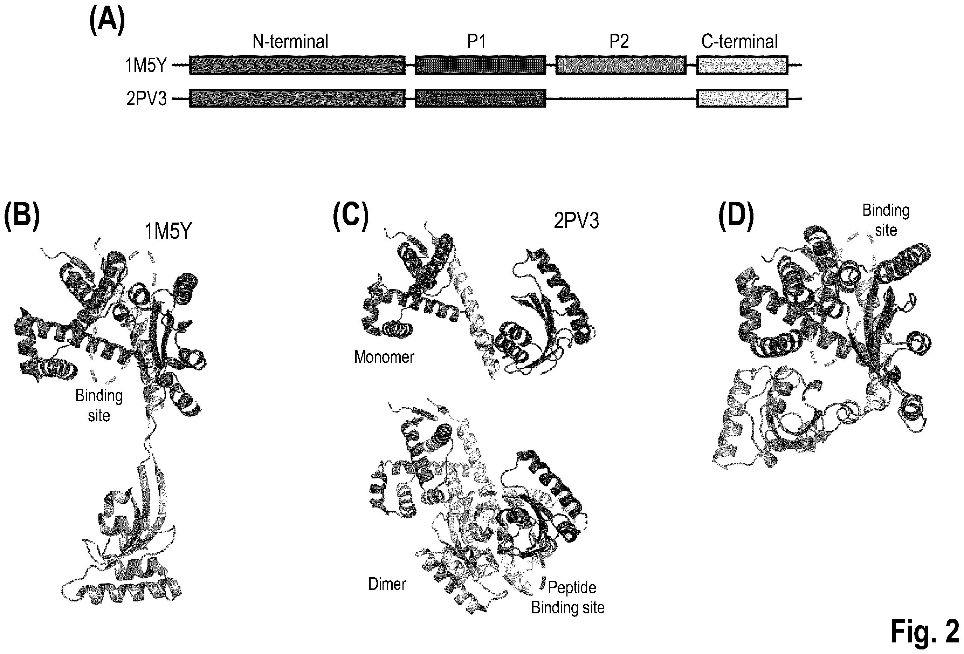

- Fig. 2:: Domain organization and structure of SurA. (A) The NC-core domain of SurA comprises the N-terminal domain (purple) and the long C-terminal helix (yellow). The two peptidyl-prolyl isomerase domains (P1, blue and P2, green) are positioned in between the N- and C-terminal domain. The X-ray structure of 1M5Y comprises the full length protein whereas the X-ray structure of 2PV3 is a SurA variant lacking the P2 domain. (B) X-ray structure of full length SurA (1M5Y) colored as in (A). The potential drugable pocket (DrugScore >0.8) is highlighted in orange. (C) X-ray structure of SurA lacking the P2 domain in presence of a helical peptide binding to the P1 domain colored as in (A). Of note, SurA might form a dimer upon peptide binding (Xu et al. 2007). The interface of the bound peptide and the SurA dimer was considered for inhibitor docking and is highlighted in red. (D) Model of the solution structure of SurA generated by extended molecular dynamics simulations (>30 µs) as identified recently (Calabrese et al. 2020; Marx et al. 2020; Jia et al. 2020). In contrast to the x-ray structure (B), P2 collapses over the N-terminal domain. Thereby P1 forms a closed version of the original proposed binding site (DrugScore >1.0, orange). This buried binding site was used for further docking and simulation studies.

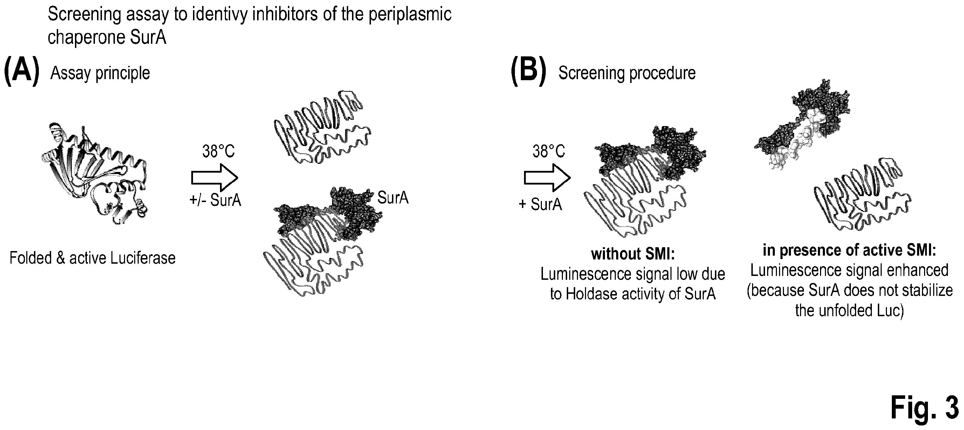

- Fig. 3:: Screening assay principle. (A) firefly Luciferase is denatured by mild heat treatment at 38.5°C. In absence of SurA, Luciferase can refold, is therefore catalytically active and hence a luminescence signal is detectable. In presence of SurA, Luciferase refolding is inhibited by the holdase activity of SurA and thus no luminescence signal is detectable. (B) SurA inhibitors allow Luciferase refolding in the presence of SurA since they inhibit the holdase activity of SurA. As a result, a luminescence signal is detected in presence of a SurA inhibitor.

### EXAMPLES

### 1. Background information: SurA biology

The periplasmic chaperone SurA is essential for shuttling outer membrane (OM) proteins across the periplasm of Gram-negative bacteria and thereby shapes the composition and barrier function of the OM. A drug inhibiting SurA will induce profound rearrangements of OM composition (which coincides with reduced serum resistance and reduced overall virulence) and integrity (which does significantly enhance susceptibility to antibiotics normally excluded by the OM and may even break resistance). A SurA inhibitor is intended to be used for supportive treatment of urinary tract, gastrointestinal or bloodstream infections with e.g. *E. coli* by promoting host-induced clearance (pathoblocker) and by sensitizing it to antibiotics (sensitizer). In the case of resistant organisms, it could be used in combination with antibiotics that are normally not effective in Gram-negatives (sensitizer) or by re-sensitizing towards drugs that were inefficient due to resistance mechanisms and can be overcome e.g. by increasing the drug influx (resistance breaker). An overview of the versatile applications of a SurA inhibitor and the impact of a SurA depletion (as a proof of concept) is given in Figure 1. The SurA inhibitor according to the invention will presumably be active against enteropathogens that share high protein sequence identity with *E. coli* SurA (i.e. *Klebsiella, Shigella, Salmonella)* but is inactive against all Gram-positives (lacking SurA). Thus, the main genera composing the gut microbiota are not affected by a SurA inhibitor and the inventors presume a reduced risk of side effects and emergence and spread of resistance mechanisms within the gut.

Apart from the effect of a lack of SurA in *E. coli* and closely related pathogens, the inventors have clear evidence for the important role of SurA also for *Pseudomonas aeruginosa* (Pa) virulence and antibiotic resistance. During previous studies the inventors analyzed the changes in OM protein abundance induced by the knockout of *surA* in *Yersinia enterocolitica.* The inventors found that not all OMPs were affected at the same extent, but OMPs harboring a high number of beta strands, amongst them LptD (an essential protein involved in LPS transport) and FhuA (an iron uptake receptor, essential for virulence), were strikingly reduced in abundance. The same holds true if SurA is depleted in *Pseudomonas aeruginosa.* Thus, SurA does not interact with a specific protein but preferably binds to a number of different OM beta barrel proteins. All of which have in common a high number of beta strands. As both LPS transport and iron uptake are essential to growth and virulence of bacterial pathogens, and proper LPS assembly and insertion is vital for the OM barrier function, the interference with both is highly desirable when thinking of application of a SurA inhibitor. By depleting SurA even multiresistant Pa could be re-sensitized to treatment with antibiotics. Given the fact that homology modelling reveals great structural similarity also with SurA of Pa a SurA inhibitor active against *E. coli* could also be active in Pa.

SurA is characterized as an ATP-independent periplasmic chaperone and has been described as a holdase. The X-ray structure of full length SurA was solved several years ago (PDB ID: 1M5Y). Of note, the structure showed an asymmetric dumbbell like structure in which the NC-core domain containing the N-terminal and C-terminal moieties of SurA (Figure 2 A+B) forms together with the first peptidyl-prolyl isomerase domain (P1) the larger side of the dumbbell. In contrast to the close association of the P1 domain with the NC-core, the P2 domain faces in the opposite direction in the crystal structure. Moreover, it was shown that the NC-core contains the chaperone activity and is sufficient to rescue SurA deletion phenotypes. The P1 domain does not display peptidyl-prolyl isomerase activity, but it was shown to interact with peptides enriched in aromatic residues which resemble OMP β-strands. Yet, the X-ray structure of SurA in complex with such peptides (PDB ID: 2PV3) was solved with a SurA lacking the P2 domain and displayed a dimeric interaction with a helical peptide (Figure 2 A+C). *In silico* analysis of the drugability of SurA identified one potential pocket in the interface between the NC/core and the P1 domain (Figure 2 B, DrugScore >0.8). Also, the peptide binding interface comprising two P1 domains was taken into consideration for inhibitor binding (Figure 2 C). Recently, the understanding of the interactions of SurA with its substrates in solution has significantly advanced. A series of studies applying crosslinking, mass spectrometry, solution techniques such as NMR spectroscopy and single molecule FRET experiments as well as computational approaches showed that SurA adopts a more closed structure in solution. Furthermore, they showed that SurA acts as a monomer and even more importantly, natural substrates interact with both the NC-core and the P1 domain. The inventors were able to simulate structures in agreement with the reported solution conformations of SurA (Figure 2 D). Of note, the previously identified druggable pocket (Figure 2 B) was affected by those solution state adjustments leading to an increase in its druggability (DrugScore >1.0). Therefore, the recent advances in the understanding of the molecular function of SurA will support our efforts to improve the design of our SurA inhibitors. However, being flexible and able to interact with many different substrates does render SurA actually not an easy target for drug development.

The inventors have developed several Target Product Profiles (TPPs; see section 5 below) where they detailed the use of a SurA inhibitor to reduce shedding time and spread of infection during gastrointestinal infection (TPP1), use the inhibitor as alternative treatment to long-term low-dose antibiotic treatment of recurrent UTI (TPP2), or enhance complement mediated lysis and host-mediated clearance during bloodstream infection, enhance antibiotic efficacy, and/or broaden the repertoire of antibiotic drugs that can be administered (TPP3).

### 2. Primary screening assays & assays used for evaluation

### 2.1 Primary assay: Luciferase-based SurA activity assay

The biological function of SurA is to bind and to keep outer membrane proteins that have reached the periplasm in an unfolded but folding-competent state. The inventors' assay is based on this interaction and uses firefly Luciferase (fLuc) as a non-native substrate. Folded, active fLuc does not interact with purified SurA. However, fLuc exhibits thermal instability and starts to unfold when heated to 38.5°C. This is exploited in the assay (Figure 3). The loss of tertiary structure of fLuc leads to the exposure of binding sites for SurA. It is known that SurA binds selectively to a special group of substrates. These substrates have in common the specific patterns of hydrophobic amino acids that are highly enriched in the beta strands of integral outer membrane proteins. These motifs are present in OMPs of all Gram-negative bacteria and in fLuc as well. Upon mild heat treatment of fLuc and its subsequent partial unfolding, fLuc is accepted as a substrate by SurA. Of note, these intermediates served as reporter substrates to determine the activity of a cytoplasmic folding chaperone. By binding to the partially unfolded fLuc, active SurA, acting as a holdase, stabilizes the unfolded, inactive form of luciferase (Figure 3 A). This interaction remains stable upon temperature reduction. Next, the temperature of the assay is reduced to room temperature and luciferase activity is subsequently measured by addition of Luciferin and ATP. In conditions without SurA, fLuc will refold into its active conformation resulting in a luminescence signal. In contrast, in presence of SurA, fLuc will be kept in its inactive unfolded conformation by the SurA holdase activity thereby resulting in a decreased luminescence signal. In the case of presence of a SurA inhibitor (Figure 3 B), fLuc will adopt an active conformation resulting in an increased luminescence signal compared to conditions without SurA inhibition.

In short, the Luciferase based SurA activity assay was performed in SurA Activity Assay Buffer (40 mM HEPES pH 7.4, 20 mM MgSO4, 0.5 g/l BSA). SurA aliquots were thawn at RT, activated by mild heat treatment and cooled down to RT for 10 min. SurA concentration was adjusted to 1.6 µM in Activity Assay Buffer (protein solution). Solution A containing 10 mM ATP as well as 0.3 mM Luciferin (Promega Beetle Luciferin, Potassium Salt) in water was prepared. Luciferase working solution was prepared by diluting 1 ml Luciferase working stock (100 nM) with 9 ml assay buffer. 33 µl of the protein solution were added to every 2nd row of a well of a 384-well PS, white, flat bottom plate. The remaining rows were filled with assay buffer serving as a control for compound induced luciferase effects. Subsequently, 2 µl of compound in DMSO were added in quadruplicates (4 wells each of protein solution and buffer controls). These compound solutions could contain either various compounds or different dilutions of the same compound allowing screening inhibitor activity in a dose dependent manner. After compound addition, plates were incubated 15 min at RT. After this incubation, 5 µl of Luciferase working solution were added to each well. Next, the plates were incubated 25 min at 38°C to partially unfold the Luciferase. Subsequently, the assay plate was cooled for 5 min at 4°C followed by 10 min incubation at RT prior to luminescence measurement. Luminescence was measured in a TECAN infinite M200 pro plate reader for each well individually after injection of 10 µl Solution A.

Of note, the SurA activity assay was performed similar at the ELF but with optimizations in regard of the higher throughput including the change of the plate format to 1536-wells. The SurA activity assay performed at the ELF contained the following steps (adapted from the QHL report provided by the ELF). First, SurA was thawed at RT and heat activated in a water bath. Next, the vial was taken out of the water bath and cooled down to RT for 30 min @ bench. Afterwards, the SurA concentration was adjusted in assay buffer to 2 µM. Noteworthy, the SurA solution was kept on ice. 15 nL of 4 mM compound in DMSO were added to columns 9-48 with Echo acoustic nano-dispenser (ECHO). Additionally, 15 nL 100% DMSO were transferred to columns 1-8 with ECHO. 3 µL assay buffer were added to columns 5-6 as well as 3 µL 2 µM SurA to columns 1-4 and 7-48 using the Certus dispenser. The plate was spun for 60 seconds at 1000 rpm to ensure proper fluid localization at the bottom of the wells. Next, the plate was incubated 15 minutes at 35°C prior to addition of 1 µL 6 nM luciferase working solution to each well of the plate using parallel dispensing with the Certus. Again, the plate was centrifuged for 60 seconds at 1000 rpm. Luciferase unfolding was performed with a two-step incubation, first for 25 minutes at 35°C followed by 25 minutes at 38.5°C. To allow refolding of the luciferase, the plate was incubated 30 minutes at RT. Finally, 2 µL substrate mix (180 µM Luciferin with 12 mM ATP) were added simultaneously to all wells using the Fluorometric Imaging Plate Reader (FLIPR) and the luminescence signal was measured immediately afterwards on the FLIPR (Gain 70,000).

### 2.2 Microscale thermophoresis

His-tagged SurA was labeled with the Monolith His-Tag Labeling Kit RED-tris-NTA (Nanotemper) according to the manufacturer's instructions.

Microscale thermophoresis measurements at the ELF were performed as follows. Compounds were tested in 8-point curves at a top starting concentration of 100 - 0.39 µM. A reference ligand (TOOL-1) was included in every run. This ligand, inhibiting SurA activity in the primary assay and biophysically interacting with SurA protein had been identified by screening own libraries in-house, not during the ELF screen. 12-point dose responses of reference compound were also set up and tested between runs. Final measurement conditions were 40 mM HEPES pH 7.4, 0.1 % Pluronic-F127, 50 nM SurA with Monolith settings - 40 % excitation and 60 % MST power. MST data (capillary scan and shape, initial fluorescence and MST traces) were analyzed and a score was given to each compound. Where at all possible, KD values have been calculated but these values must be looked at in conjunction with the compound score. For example, compounds which have been ranked highly (score 1 or 2) may have a lower pKd value than some which have been ranked with a score of 3.

Microscale thermophoresis measurements were performed in 40 mM HEPES pH 7.4, 0.05 % Tween-20 at 25°C with a Monolith NT.115 (NanoTemper) in standard capillaries. KD measurements consisted of 8-16 MST measurement points that were acquired with 50 nM His-labeled SurA in the presence of 2500 - 0.076 µM compound in presence of 5 % DMSO and were performed in triplicates.

### 2.3 Nano differential scanning fluorimetry

Nano Differential Scanning Fluorimetry (nanoDSF) measurements of the QHL (qualified hit list) compounds provided by the ELF were performed on a Tycho system (NanoTemper). Each QHL compound was tested in triplicates at 100 µM in presence of 2 µM SurA in 40 mM HEPES pH 7.4, and a previously identified reference ligand (TOOL-1) was included as a positive control at 333 µM.

nanoDSF thermal shift assays of resynthesized QHL compounds as well as of QHL-10 and QHL-24 (QHL-24 is a preferred embodiment of the compound according to the invention) shelf derivatives were performed in 40 mM HEPES pH 7.4, 150 NaCl as well as in 50 mM HAc pH 5. To this end, 5 µl of a 2 mM compound solution in DMSO were mixed with 95 µl of a 20 µM SurA solution. Technical triplicate measurements were performed on a Prometheus system (NanoTemper).

### 2.4 Compound docking

Initially, QHL compounds were docked into two available SurA structures: the full-length protein (1M5Y) or SurA mutant lacking the PPI2 domain (2PV3). The lack of PPI2 allows SurA to establish a dimeric conformation, where a small peptide resides in an interface between the PPI1 domains. Full-length SurA displays a potentially drugable pocket (DrugScore >0.8, calculated using SiteMap program) composed by the N- and C-termini and the PPI1 domain, which was used for initial docking studies. Additionally, the peptide interaction interface of SurA ΔPPI2 was considered for docking. Of note, these pockets do not share the same residues. Flexible docking was performed using the induced-fit protocol (Glide), where protein-ligand complexes (herein called poses) were ranked by docking score. The best docking poses were selected by visual inspection of their protein-ligand interactions among the highest-ranked complexes. Selected poses underwent molecular dynamics simulations in an explicit solvent environment for 200 ns, using Desmond engine and the force-field OPLS3e. The frequency of the protein-ligand interactions along the simulations was used as criterion to determine the pose stability and quality of the docking.

Due to advances in the understanding of the details of the interaction of Su-rA with its substrates, further docking studies of SurA with compounds QHL-24 (preferred embodiment of the compound according to the invention) and QHL-10 as well as their respective libraries were performed with a model of the solution structure of SurA displaying a more collapsed conformation, as identified recently. This model was generated by comprehensive evaluation of SurA dynamics in explicit solvent, using longer molecular dynamics simulations (>30 µs). The inventors observed that the collapse of PPI2 over the N-terminal α-helices generated a buried version of the original proposed binding site (DrugScore >1.0, calculated using SiteMap program) which has been used now to propose new derivatives.

### 2.5 Minimal inhibitory concentration assay

The intended effect of a SurA inhibitor should be the induction of a disturbance of the outer membrane composition resulting in an increased susceptibility to antibiotic treatment. Of note, Vancomycin is typically not used to treat infections with Gram-negative bacteria, because it is excluded by the OM and not efficient at concentrations that could be achieved in patients. However, at appropriate dosing *in vitro* it can be used to detect outer membrane defects. For determining a potential sensitizing effect of Vancomycin treatment, the inventors first tested whether the compounds at hand were bactericidal *per se.* Therefore, the inventors performed a minimal inhibitory concentration (MIC) assay. To this end, 5 ml overnight cultures of E. *coli* BW25113 (Keio-Collection reference strain), *E. coli* JW2496-3 (Keio-Collection single gene knockout for *bamB,* which suffers from decreased outer membrane integrity) and E. *coli ATCC* 25922 (a reference strain recommended for antibiotic susceptibility testing) were grown in Mueller Hinton Broth, Cation adjusted (MHB-II). The next day, the OD was adjusted to obtain 1* 10⁸ CFU/ml using fresh MHB-II. Next, this bacterial solution was diluted 1:100 into 20 ml fresh MHB-II. 50 µl of this bacteria solution were then transferred into each well of a 96-well plate prefilled with 50 µl MHB-II supplemented with the desired compounds. The test plates for the bactericidal assessment contained dilution series of the compounds from 100 - 1.7 µM. Plates were sealed with a gas permeable membrane and incubated o/n at 37°C in a humid chamber without shaking. After o/n incubation, bacterial growth was quantified by measuring the optical density at 600 nm wavelength in a multiplate reader. Compounds showing no growth inhibition at 100 µM were considered nonbactericidal. Of note, the bactericidal effect of Vancomycin was assessed similarly for each test strain using Vancomycin concentrations ranging from 512 - 0.25 mg/ml. This was done to identify a Vancomycin concentration at which the strains just managed to survive.

To test for the sensitizer properties of our compounds, the inventors then performed a sensitizer MIC assay. To this end, *E.coli* ATCC 25922 inocula were prepared as described above. Each well of a 96-well plate was prefilled with a fixed compound concentration of 100 µM and a dilution series of Vancomycin ranging from 512 - 2 mg/ml. A sensitizing effect was defined as reduction of the MIC value of Vancomycin.

### 2.6 Cytotoxicity assay

Cell cytotoxicity was evaluated monitoring the LDH release from HepG2 cells using the CytoTox 96^{®} Non-Radio Cytotoxicity Assay (Promega) according to the manufacturer's instructions. In short, 7500 HepG2 cells/well in 50 µl FCS-free RPMI-1640 medium were incubated 1 h at 37°C in presence of 500 µM compounds. After incubation, 25 µl were transferred into a fresh 96-well flat clear bottom plate and mixed with 25 µl CytoTox 96^{®} Reagent. LDH release reactions were incubated 30 min at RT in the dark and 25 µl Stop Solution were added. LDH release was measured at 490 nm in a multiplate reader.

### 3. Top 10 of the qualified hit list (QHL)

### 3.1 QHL

The following paragraphs describing the screen at the European Lead Factory (ELF) to generate the QHL were taken from the QHL report provided by the ELF.

Starting with approximately 400,000 compounds, a set of 3991 compounds was left for testing in dose response experiments. Due to acquisition of a lower assay window for part of the data obtained during the screening campaign of ELFSC17_02, the data-set was split into P1 and P2 for analysis in ActivityBase. For P1 the initially set criterion of Z-score ≥ 4 (~ 4%) was used for selection of actives and for P2 this selection criterion was adjusted to effect ≥ 40% (Z-score -15). The primary screen (P1 + P2) resulted in 4205 actives in total, of which 3558 actives with a Z-score > 4 (P1) and 647 actives with a percentage effect ≥ 40% (P2). In order to reduce this total number of 4205 compounds to a maximum of 4000 compounds allowed to be ordered in serial dilutions for dose response experiments (i.e. 1% of 400,000), the criterion for P1 was changed to Z-score > 4.3 gaining 3344 compounds. Finally, this resulted in the selection of 3991 compounds (i.e. 3344 + 647) for active confirmation in serial dilution experiments (C2). The confirmed actives of ELF program ELFSC17_02 were tested as serial dilutions in the primary assay and deselection assay to generate dose response curves (DRCs). The serial dilutions consisted of 7 equidistant concentration steps (20 µM to 27.4 nM) and were tested in the primary and in the deselection assay in duplo (n=2) in 1536-well format. In the active confirmation in DRC-format 315 compounds gave a pEC50 > 4.7 with a Hill slope between 0.24 and 29.12. None of these 315 compounds had a pEC50 of >7.6 or were added based on the Bayesian model. For the luciferase interference deselection assay in DRC-format, luciferase was left out in the Max effect wells, mimicking a 100% effect situation and resulting in a decreased signal. Compounds showing a signal decrease larger than 5 times the standard deviation (Z-score > 5) were identified as luciferase inducer. This resulted in the deselection of one compound which was not previously confirmed in C2. The ActivityBase application (IDBS) was used to analyze the data and to calculate quality parameters like Z-prime and Signal to Background ratio (S/B). For all compounds the % effect was calculated for all seven concentrations. ActivityBase was also used to calculate the pEC50 and Hill Slope. For compounds that did not reach 50% effect at 20 µM no curve could be fitted and the Hill Slope was not calculated (pEC50 <4.7). The same was the case for compounds that showed 100% effect at 20 µM but did not descent to <50% at 27 nM (pEC50 >7.6). For compounds where no curve was fitted but reached >50% effect at 20 µM the Fit_Max was locked at 100% effect. For compounds where no curve was fitted but reached 100% effect at 20 µM and descended to <50% at 27 nM the Fit_Min was locked at 0%. This data will be used for the selection of compounds towards the preliminary hit list (PHL) and subsequent qualified hit list (QHL). Based on the results of C2 and D1 a list of 383 compounds was created as the preliminary hits, which were evaluated with MST (O1). PCT reviewed the compounds on the basis of their activity, structures physicochemical properties and purity according to LCMS analysis to finally create an RHL of 53 compounds. Following legal clearance and implementation of further prioritization a QHL of 50 compounds was created (Table 1).

**Table 1: Qualified Hit List provided by the ELF. Compounds are sorted ascending according to their plC50 in the C2 assay. The table includes the key data from Luciferase assay P1 screening the ELF library, from the dose response Luciferase assay C2, the deselection assay D1 from Luciferase assay (Luciferase interference) and MST measurements O1, as well as calculated properties. The results from the MST assay O1 is given as plC50 values and as a score 1-6, with 1=binder and 6=non-binder, nt = not tested. For all compounds the sample was found to be ≥ 80% pure by UV area% at 254 nm with an m/z consistent with the proposed structure.**

| | | | | | | | | | **Assay data** | | | | | |
|---|---|---|---|---|---|---|---|---|---|---|---|---|---|---|
| **Calculated properties** | | | | | | | | | **Primary (P1)** | **assay** | **DRC (C2)** | **Deselection (D1)** | **MST** | |
| **Compound** | **HBA** | **HBD** | **ALogP** | **cLogD** | **MW** | **QED** | **TPSA** | **LipE** | **Z-score** | **% Effect** | **plC50** | **plC50** | **score** | **plC50** |
| **QHL-01** | 10 | 4 | 2.21 | 2.21 | 479.53 | 0.48 | 143.55 | 4.1 | 75.490 | 67 | 6.31 | 7.250 | 2 | =4.360 |
| **QHL-02** | 5 | 2 | 4.05 | 2.58 | 390.52 | 0.74 | 132.82 | 2.08 | 250.300 | 24 | 6.13 | 5.260 | 6 | <4.000 |
| **QHL-03** | 8 | 2 | 3.71 | 3.71 | 416.43 | 0.50 | 98.51 | 2.41 | 697.940 | 491 | 6.12 | <4.700 | 4 | <4.000 |
| **QHL-04** | 7 | 3 | 3.97 | 2.50 | 443.47 | 0.41 | 136.63 | 2.1 | 229.080 | 231 | 6.07 | <4.700 | 6 | <4.000 |
| **QHL-05** | 9 | 3 | 1.77 | 1.77 | 450.88 | 0.53 | 116.32 | 4.28 | 260.420 | 231 | 6.05 | 4.810 | 4 | <4.000 |
| **QHL-06** | 5 | 2 | 3.46 | 3.46 | 303.31 | 0.61 | 70.92 | 2.57 | 174.89 | 342 | 6.03 | 4.970 | 5 | <4.000 |
| **QHL-07** | 5 | 1 | 3.26 | 3.26 | 338.43 | 0.79 | 96.01 | 2.76 | 424.650 | 47 | 6.02 | 4.920 | 3 | =4.470 |
| **QHL-08** | 7 | 2 | 5.2 | 3.72 | 464.47 | 0.36 | 101.66 | 0.77 | 156.91 | 307 | 5.97 | <4.700 | 6 | <4.000 |
| **QHL-09** | 5 | 3 | 3.42 | 3.41 | 380.46 | 0.61 | 106.67 | 2.53 | 127.870 | 23 | 5.95 | <4.700 | 6 | <4.000 |
| **QHL-10** | 9 | 5 | 3 | 1.53 | 470.48 | 0.30 | 147.39 | 2.89 | 100.520 | 116 | 5.89 | 5.160 | 1 | =4.400 |
| **QHL-11** | 8 | 2 | 3.12 | 1.66 | 432.43 | 0.44 | 110.89 | 2.7 | 315.490 | 49 | 5.82 | 5.140 | 4 | <4.000 |
| **QHL-12** | 8 | 1 | 2.24 | 2.24 | 468.93 | 0.62 | 83.88 | 3.55 | 186.320 | 205 | 5.79 | 5.250 | 4 | <4.000 |
| **QHL-13** | 7 | 3 | 2.96 | 2.96 | 404.46 | 0.54 | 92.35 | 2.82 | 288.750 | 51 | 5.78 | 4.860 | 6 | <4.000 |
| **QHL-14** | 9 | 3 | 3.2 | 1.72 | 479.55 | 0.51 | 151.45 | 2.56 | 325.950 | 211 | 5.76 | <4.700 | 3 | <4.000 |
| **QHL-15** | 9 | 5 | 2.88 | 1.41 | 436.46 | 0.38 | 147.39 | 2.76 | 220.430 | 292 | 5.64 | 4.760 | 5 | <4.000 |
| **QHL-16** | 8 | 2 | 3.1 | 1.00 | 486.56 | 0.56 | 83.14 | 2.47 | 49.100 | 42 | 5.57 | <4.700 | 4 | <4.000 |
| **QHL-17** | 4 | 1 | 2.72 | 3.17 | 251.28 | 0.73 | 50.80 | 2.82 | 60.940 | 85 | 5.54 | 4.720 | 4 | <4.000 |
| **QHL-18** | 7 | 2 | 4.2 | 2.75 | 430.45 | 0.44 | 101.66 | 1.32 | 230.92 | 588 | 5.52 | 4.790 | 3 | =4.610 |

| | **Calculated properties** | | | | | | | | **Prima(P1)** | **assay** | **DRC (C2)** | **Deselection (D1)** | **MST** | |
|---|---|---|---|---|---|---|---|---|---|---|---|---|---|---|
| **Compound** | **HBA** | **HBD** | **ALogP** | **cLogD** | **MW** | **QED** | **TPSA** | **LipE** | **Z-score** | **% Effect** | **plC50** | **plC50** | **score** | **plC50** |
| **QHL-19** | 3 | 1 | 3.56 | 3.56 | 285.36 | 0.80 | 70.59 | 1.96 | 42.020 | 74 | 5.52 | <4.700 | 6 | <4.000 |
| **QHL-20** | 4 | 2 | 3.67 | 3.66 | 318.40 | 0.61 | 92.93 | 1.82 | 151.240 | 168 | 5.49 | 5.170 | 3 | =4.660 |
| **QHL-21** | 4 | 1 | 4.36 | 4.37 | 319.40 | 0.78 | 46.92 | 1.12 | 25.750 | 49 | 5.48 | 4.710 | 4 | <4.000 |
| **QHL-22** | 8 | 3 | 4.51 | 4.51 | 432.47 | 0.48 | 108.46 | 0.96 | 94.040 | 124 | 5.47 | <4.700 | 6 | <4.000 |
| **QHL-23** | 6 | 1 | 2.55 | 2.56 | 341.43 | 0.79 | 90.77 | 2.92 | 103.860 | 56 | 5.47 | 4.950 | 4 | =4.480 |
| **QHL-24** | 7 | 2 | 4.06 | 4.22 | 410.47 | 0.46 | 78.84 | 1.39 | 191.970 | 151 | 5.45 | 4.420 | 1 | =4.280 |
| **QHL-25** | 4 | 1 | 4.74 | 4.74 | 360.43 | 0.57 | 79.45 | 0.7 | 162.110 | 98 | 5.44 | <4.700 | 6 | <4.000 |
| **QHL-26** | 7 | 4 | 4.25 | 4.25 | 422.44 | 0.35 | 107.10 | 1.19 | 139.790 | 256 | 5.44 | 4.770 | 3 | =4.430 |
| **QHL-27** | 6 | 3 | 2.96 | 1.49 | 298.34 | 0.73 | 87.14 | 2.43 | 238.580 | 30 | 5.39 | 4.680 | 6 | <4.000 |
| **QHL-28** | 6 | 2 | 4.16 | 2.70 | 384.38 | 0.51 | 92.43 | 1.15 | 147.19 | 516 | 5.31 | <4.700 | 3 | =5.230 |
| **QHL-29** | 5 | 0 | 3.54 | 3.54 | 305.33 | 0.58 | 53.08 | 1.75 | 244.440 | 21 | 5.29 | 4.680 | 6 | <4.000 |
| **QHL-30** | 7 | 1 | 2.24 | 2.24 | 343.33 | 0.84 | 83.09 | 3.02 | 91.620 | 106 | 5.26 | 4.800 | 3 | <4.000 |
| **QHL-31** | 5 | 1 | 3.21 | 3.21 | 314.36 | 0.80 | 88.69 | 2.02 | 113.59 | 431 | 5.23 | 5.050 | 3 | =4.070 |
| **QHL-32** | 5 | 0 | 3.88 | 3.88 | 348.42 | 0.56 | 81.06 | 1.34 | 170.08 | 1080 | 5.22 | <4.700 | 6 | <4.000 |
| **QHL-33** | 6 | 0 | 3.42 | 0.91 | 449.59 | 0.72 | 45.25 | 1.78 | 23.590 | 16 | 5.2 | <4.700 | 3 | =5.290 |
| **QHL-34** | 4 | 0 | 4.11 | 2.71 | 448.50 | 0.71 | 58.08 | 1.04 | 83.910 | 169 | 5.15 | <4.700 | nt | nt |
| **QHL-35** | 8 | 2 | 3.13 | 2.69 | 472.32 | 0.59 | 96.44 | 2.01 | 140.290 | 14 | 5.14 | <4.700 | nt | nt |
| **QHL-36** | 5 | 1 | 2.84 | 2.84 | 344.79 | 0.93 | 58.64 | 2.27 | 51.400 | 7 | 5.11 | <4.700 | 6 | <4.000 |
| **QHL-37** | 3 | 1 | 3.85 | 3.85 | 285.36 | 0.79 | 70.59 | 1.26 | 144.990 | 154 | 5.11 | <4.700 | 6 | <4.000 |
| **QHL-38** | 4 | 2 | 3.32 | 3.30 | 311.37 | 0.89 | 50.72 | 1.77 | 29.800 | 23 | 5.09 | <4.700 | 4 | =4.760 |
| **QHL-39** | 8 | 6 | 1.48 | 1.57 | 361.42 | 0.63 | 168.44 | 3.61 | 48.840 | 54 | 5.09 | <4.700 | 6 | <4.000 |
| **QHL-40** | 5 | 3 | 2.19 | 2.19 | 332.42 | 0.78 | 106.67 | 2.9 | 103.750 | 137 | 5.09 | <4.700 | 6 | <4.000 |
| **QHL-41** | 5 | 2 | 2.73 | 2.73 | 331.37 | 0.77 | 71.09 | 2.35 | 217.660 | 205 | 5.08 | <4.700 | 4 | =4.480 |
| **QHL-42** | 6 | 0 | 3.86 | 3.86 | 378.45 | 0.53 | 90.30 | 1.19 | 32.06 | 125 | 5.05 | <4.700 | 6 | <4.000 |
| **QHL-43** | 5 | 2 | 3.12 | 3.12 | 340.42 | 0.81 | 67.43 | 1.9 | 79.320 | 47 | 5.02 | 4.880 | nt | nt |
| **QHL-44** | 7 | 1 | 2.98 | 2.81 | 440.92 | 0.66 | 66.81 | 2.03 | 377.460 | 47 | 5.01 | 4.380 | nt | nt |
| **QHL-45** | 6 | 1 | 2.44 | 0.97 | 369.41 | 0.85 | 76.07 | 2.55 | 16.330 | 26 | 4.99 | 4.490 | 4 | =4.040 |
| **QHL-46** | 6 | 2 | 0.16 | 0.16 | 261.28 | 0.76 | 70.67 | 4.69 | 18.980 | 3 | 4.85 | <4.700 | 3 | =4.950 |
| **QHL-47** | 6 | 2 | 4.5 | 4.51 | 405.83 | 0.53 | 76.24 | 0.3 | 107.120 | 101 | 4.8 | <4.700 | nt | nt |
| **QHL-48** | 5 | 1 | 3.85 | 3.85 | 327.37 | 0.78 | 64.63 | 0.91 | 49.450 | 6 | 4.76 | <4.700 | nt | nt |
| **QHL-49** | 6 | 2 | 3.53 | 3.53 | 384.47 | 0.61 | 76.66 | 1.22 | 31.630 | 4 | 4.75 | <4.700 | nt | nt |
| **QHL-50** | 6 | 1 | 4.57 | 4.57 | 369.29 | 0.72 | 73.86 | 0.15 | 12.040 | 30 | 4.72 | <4.700 | nt | nt |

### 3.2 Re-evaluation of QHL

After receiving the QHL (Table 1) and the QHL compound remainders in DMSO, the inventors re-evaluated the provided compounds. To this end, the inventors conducted the primary assay (2.1), MST measurements (2.2), nanoDSF measurements (2.3) as well as docking studies (2.4). The compound ranking emphasized at this stage the performance in the primary assay including the Luciferase induction in absence of SurA as a deselection criteria together with the biophysical characterization of the SurA:compound interaction by MST and nanoDSF. Furthermore, docking of the compound into the x-ray structure (1M5Y, Figure 2B) was included as an additional selection criterion, e.g. if other data were not available or inconclusive. Additionally, the data generated at the ELF was considered to allow us to judge the overall performance of the compounds. This evaluation process led to a ranking of the QHL compounds (Table 2). Out of the 50 QHL compounds, the five overall best performing candidates (QHL-10, QHL-18, QHL-24 (preferred embodiment of the compound according to the invention), QHL-30, QHL-31) were chosen for resynthesis. Of note, QHL-14 was selected due to its unusual performance in the nanoDSF measurement in which it decreased both inflection points of SurA.

**Table 1: Top 10 compounds after re-evaluation. Compound ranking is based on the overall performance in the re-evaluation process including nanoDSF, MST, activity in the primary assay, docking studies in conjunction with the data of the QHL.**

| **ID** | **Ranking** | **Selected re-synthesis/ commercial** | **Re-eva nanoDSF luation (Tuebingen) (Tycho)** | | **MST** | **Primary Assay Activitiy** | **docking 1m5y** | **ELF Prima(P1) assay(P1)** | | **DRC (C2) plC50** | **Deselection (D1) plC50** | **MST score** | **plC50** |
|---|---|---|---|---|---|---|---|---|---|---|---|---|---|
| | | | **Ti#1** | **Ti#2** | **MST score** | | | **Z-score** | **%Effect** | | | | |
| **QHL-14** | 1 | x | - | - | 2 | - | unstable | 325.95 | 211 | 5.76 | <4.700 | 3 | <4.000 |
| **QHL-10** | 2 | re-synthesis | ? | + | 2 | + | unstable | 100.52 | 116 | 5.89 | 5.16 | 1 | 4.4 |
| **QHL-18** | 3 | re-synthesis | ? | ? | 2 | + | unstable | 230.92 | 588 | 5.52 | 4.79 | 3 | 4.61 |
| **QHL-24** | 4 | re-synthesis | none | + | inconclusive | + | stable | 191.97 | 151 | 5.45 | 4.42 | 1 | 4.28 |
| **QHL-30** | 5 | re-synthesis | + | + | inconclusive | + | unstable | 91.62 | 106 | 5.26 | 4.8 | 3 | <4.000 |
| **QHL-31** | 6 | x | none | + | 4 | + | stable | 113.59 | 431 | 5.23 | 5.05 | 3 | 4.07 |
| **QHL-26** | 7 | not passed | + | - | 5 | + | stable | 139.79 | 256 | 5.44 | 4.77 | 3 | 4.43 |
| **QHL-33** | 8 | not passed | none | + | 6 | + | unstable | 23.59 | 16 | 5.2 | <4.700 | 3 | 5.29 |
| **QHL-46** | 9 | not selected | none | + | nt | + | unstable | 18.98 | 3 | 4.85 | <4.700 | 3 | 4.95 |
| **QHL-47** | 10 | not selected | ? | ? | inconclusive | + | stable | 107.12 | 101 | 4.8 | <4.700 | nt | nt |

### 3.3 Evaluation of resynthesized compounds

Of the top scoring QHL compounds after re-evaluation (Table 2), the six best performing compounds QHL-10, QHL-14, QHL-18, QHL-24 (preferred embodiment of the compound according to the invention), QHL-30, QHL-31) were chosen for resynthesis or bought commercially if they were available at high purity (≥95 %).

The evaluation (Table 3) of the resynthesized/repurchased compounds included the primary assay (2.1) screening in a dose response up to a maximum compound concentration of 250 µM. Moreover, MIC assays (2.5) determining the reduction of the effective Vancomycin MIC in presence of 100 µM compound, biophysical interaction studies including MST measurement (2.2) and nanoDSF (2.3). All compounds tested showed a maximum SurA inhibition (ICₘₐₓ) at 50 µM or below. Out of the six tested QHL-10, QHL-18 and QHL-24 displayed the lowest ICₘₐₓ. Of those three, all showed binding in the MST experiments, but MST data quality was only sufficient to fit the K_{D} of QHL-24 in the range of 20-30 µM. Of those three, QHL-10 and QHL-24 (preferred embodiment of the compound according to the invention) affected the thermal stability of SurA as detected by nanoDSF. Additional docking studies (2.4) using the improved SurA solution model revealed a stable interaction of QHL-10 and QHL-24 in the interface of the NC-core and P1 domains. Therefore, QHL-10 and QHL-24 were chosen for further development, with QHL-24 being an embodiment of the compound according to the invention. To this end, the maximum available chemical space was assessed using commercially available but unfortunately only rather distantly related compounds.

**Table 2: Evaluation of resynthesized QHL compounds. * The tool compound used as positive control showed bactericidal behavior when used at 100 µM in the MIC assay and was therefore used at 12.5 µM. nt: not tested. An increase of the inflection points in the nanoDSF experiments is indicated by (+), a decrease by (-), no changes with (none) and ambiguity with a question mark (?), respectively.**

| **ID** | **Primary Assay ICmax** | **MIC (Vanco, mg/ml)** | **MST** | **nanoDSF Ti#1** | **pH Ti#2 5** | **nanoDSF Ti#1** | **pH 7.4 Ti#2** | **Docking 1M5Y** | **"closed"** |
|---|---|---|---|---|---|---|---|---|---|
| **QHL-14** | 50 µM | 512 | binding | + | + | none | none | Unstable | nt |
| **QHL-10** | 5-10 µM | 256 | binding | + | - | none | - | Unstable | stable |
| **QHL-18** | 5-10 µM | 256 | binding | ? | ? | ? | ? | Unstable | nt |
| **QHL-24** | 5-10 µM | 256 | 20-30 µM | none | + | none | - | Stable | stable |
| **QHL-30** | 10-20 µM | 256 | nt | - | none | none | - | Unstable | nt |
| **QHL-31** | 50 µM | nt | nt | nt | Nt | Nt | nt | Stable | nt |
| **TOOL-1** | 40 µM | 256* | 42 µM | + | + | none | - | Nt | nt |
| **DMSO** | n/d | 512 | n/d | | | | | | |

### 4. Compound derivatives

### 4.1 QHL-24 and QHL-10 shelf derivatives

In order to explore the commercially available chemical space around QHL-24 (preferred embodiment of the compound according to the invention) and QHL-10, the inventors ordered four (QHL-24-1 - QHL-24-4) and five (QHL-10-1 - QHL-10-5) relatives of QHL-24 and QHL-10 for further evaluation, respectively. Actually, the available relatives were rather distant to QHL-24 and QHL-10. The evaluation of these shelf derivatives included an estimate of their solubility based on the turbidity (OD600nm) as well as their activity in the primary assay (2.1), their KD (2.2), their MIC reduction of Vancomycin (2.5) and their cytotoxicity (2.6) (Table 4). Of note, almost all derivatives were more soluble compared to their parent compounds. QHL-24 displayed a poor solubility hampering almost all assays. In addition, QHL-10-3 was insoluble in DMSO and could therefore not be tested. Testing the shelf derivatives for their activity in the Luciferase-based SurA activity assay did not indicate any SurA inhibition. This was confirmed additionally by their inability to reduce the MIC of Vancomycin as well as their reduced KD values in the MST measurements. Moreover, their cytotoxicity was not superior compared to the parental compounds. Hence, the shelf derivatives tested did not yield any improvements with regard to activity or cytotoxicity. However, due to their inability to improve the properties of the parental compounds, the inventors can at least estimate the boundaries of our chemical space. Hence, the shelf-derivatives provide valuable input for the design of the QHL-24 and QHL-10 libraries.

As at the same time the evaluation of the shelf derivatives of QHL-10 and QHL-24 had been finished, new insights into the interactions of SurA with its substrates and its conformation in solution were published. Therefore, the inventors performed additional docking studies based on these new insights. Molecular dynamics simulation studies of our collaboration partners actually showed similar conformations to the published data. The inventors are currently evaluating whether the existence of these conformations holds true *in vitro* and *in vivo* by wet-lab experiments in the inventors' lab. Interestingly, docking of QHL-10 into this "closed" SurA conformation yielded a much more stable interaction as with the open structure represented by 1M5Y (Figure 2 D, Table 3).

**Table 3: Evaluation of QHL-24 and QHL-10 shelf derivatives. The estimate of solubility is based on the turbidity (OD₆₀₀ₙₘ) of a 100 µM solution in SurA Activity Assay Buffer with 5 % DMSO (OD₆₀₀ₙₘ < 0.01 = +++; 0.01 < OD₆₀₀ₙₘ <0.1 = +; OD₆₀₀ₙₘ> 0.1 = -). The activity in the primary assay is distinguished into influence on SurA (SurA inhibition = +; no SurA inhibiton = 0) or interference with Luciferase per se (no influence on Luc activity = 0; enhances Luc activity = +; reduces Luc activity = -). The MIC assay determined the reduction of the MIC for vancomycin of the E. coli ATCC 25922 strain below 256 mg/l at a compound concentration of 100 µM (yes/ no) or a bactericidal effects (b). n/a: not applicable.**

| **ID** | **MW** | **Tanimoto similarity** | **Solubility** | **primary SurA** | **assay Luciferase** | **MIC** | **MST (Kd in mM)** | **CytoTox** |
|---|---|---|---|---|---|---|---|---|
| **QHL-24** | 410.470 | | - | + | - | yes | 0.03-0.02 | precipitate (7.87%) |
| **QHL-24-1** | 345.406 | 0.818 | + | 0 | 0 | no | n/a | precipitate (20.99%) |
| **QHL-24-2** | 279.343 | 0.805 | + | 0 | - | no | 23 | 23.55% |
| **QHL-24-3** | 279.343 | 0.786 | +++ | 0 | 0 | no | n/a | 4.01% |
| **QHL-24-4** | 346.394 | 0.800 | + | 0 | 0 | no | 1.02 | precipitate (41.17%) |
| **QHL-10** | 470.490 | | - | + | - | yes | binding | 50.23% |
| **QHL-10-1** | 383.455 | 0.551 | +++ | 0 | - | no | 0.45 | 60.11% |
| **QHL-10-2** | 347.418 | 0.627 | +++ | 0 | - | no | 1.28 | 67.31 % |
| **QHL-10-3** | 321.336 | 0.638 | n/a | n/a | n/a | n/a | n/a | n/a |
| **QHL-10-4** | 344.415 | 0.642 | + | 0 | 0 | no | 6.12 | 44.15% |
| **QHL-10-5** | 341.371 | 0.593 | + | 0 | 0 | no | 0.58 | 42.16% |
| **TOOL-1** | 441.460 | | +++ | + | + | b | 0.042 | 40.85% |

### 4.2 Elaboration of hit series

Along with the evaluation of the shelf derivatives, the preparations for the synthesis and exploration of the possible chemical space around QHL-10 and QHL-24 (preferred embodiment of the compound according to the invention) were initiated. An alternative synthesis route for QHL-10 and QHL-24 was already evaluated to increase the yield of synthesis. As the following step in developing derivatives for best leads, the inventors have used information from MD simulations to suggest regions of the compound for chemical modification. Compound moieties involved in stable interactions along the analyzed trajectories were suggested as relevant. These moieties are candidates for bio-isosterism, meaning changing the functional group but keeping the similar property, to increase affinity. Alternatively, regions, where stability was not observed, became candidates for scaffold hopping, modifying the entire functional group.

Both processes were employed in a combinatorial fashion to generate a large library of derivatives. Information regarding the synthetic viability of the basic changes was provided by the inventors and included in the library generation. Due to the solubility issues observed in the previous experiments, whenever possible, functional groups with an increased sp3 carbon ratio were selected, to increase the 3D property of the compound. Library derivatization of this nature can easily reach the number of millions of possible combinations. Hence, as a prioritization strategy the inventors use clustering based on compound similarity followed by docking and simulations. The inventors understand the lead optimization step as an iterative process where data from both the synthetically viability and experimental validation would feed the library design and selection of the hits proposed in the next iteration.

### 4.3 Chemical synthesis of the compound according to the invention

The compounds of the present invention may by synthesized according to the following reaction scheme 1.

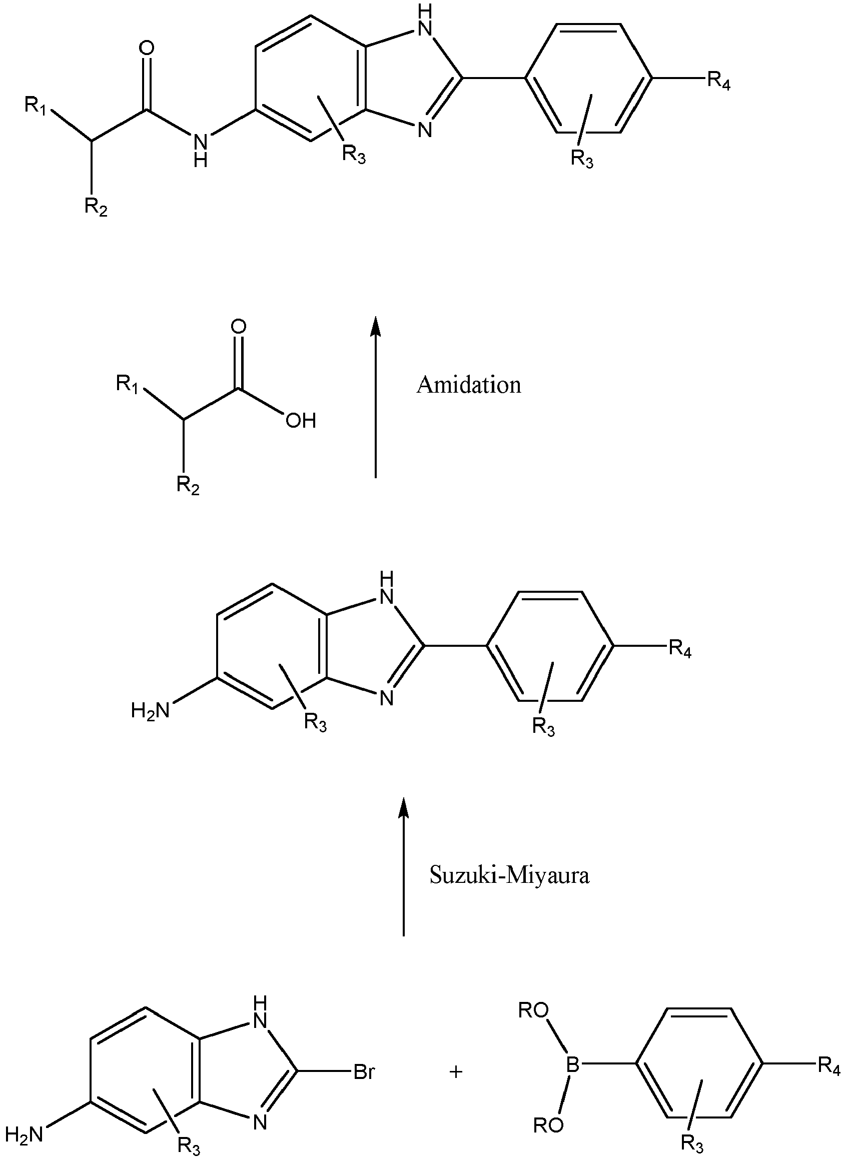

It is well known in the art that the Suzuki-Miyaura reaction is based on the Pd-catalysed coupling of an aryl- or vinyl halogenide with an aryl- or vinyl- boronic acid.

For instance, the Suzuki-Miyaura reaction may be carried out by dissolving 1 equivalent of the compound of the formula (II) and about 1.01 to 1.25 equivalents, such as 1.05 to 1.15 eq., or 1.08 to 1.11 eq., or 1.09 to 1.10 eq., of the compound of the formula (III), and a base, preferably a tertiary amine, such as triethylamine, preferably about 10 to 15 eq., such as 12.2 eq., in a mixture of an organic solvent, such as 1,4-dioxane, and H₂O (preferably in a relationship of about 3-5 : 1, such as 3.5 - 4.5: 1, or 4 : 1). About 0.2 to 0.5 eq., such as 0.25 to 0.4 eq., 0.3 to 0.35 eq. or 0.33 to 0.34 eq., of a palladium compound, preferably Pd(dppf)Cl₂, are added to the reaction mixture and stirred for an elongated period, such as 12 to 60 hours, such as 24 to 48 h, or 40 h, at a temperature affording reflux of the solvent, such as about 95°C or more, such as 100 to 110°C. The reaction mixture may by purified according to the skilled persons knowledge, e.g. by filtering, such as filtering through a solid filter, e.g., a celite material.

The amidation may be carried out by dissolving the compound of the formula (IV) and (CHR₁R₂)COR" in an amount of 0.7 to 1.3 eq. compound of the formula (IV) and 1 eq. of (CHR₁R₂)COR", preferably 1.2 eq. compound of the formula (IV) and 1 eq. of (CHR₁R₂)COR", in an organic solvent, such as acetonitrile. Approx. 1.0 to 1.4 eq., preferably 1.2 eq., of a carbodiimide, such as 1-Ethyl-3-(3-dimethylaminopropyl)carbodiimidehydrochloride (EDC-HCI), and approx. 1.0 to 1.4 eq., preferably 1.2 eq., of an alcohol hydrate, such as HOBt hydrate, are added. The reaction mixture may be stirred for, e.g., 24 to 48 hours, such as 40 h, at a temperature of, e.g., about 30 to 50°C, such as 40°C. The reaction mixture may by purified according to the skilled persons knowledge, e.g. by subjecting the reaction mixture to High Performance Counter Current Chromatography or High Performance Liquid Chromatography.

It will be appreciated that other synthesis methods may be used as well.

In addition, the synthesis may require the use of a protecting group. In general, a "protecting group" or a "protective group" is a reversibly formed derivative of an existing functional group in a molecule. The protective group is temporarily attached to decrease reactivity so that the protected functional group does not react under synthetic conditions to which the molecule is subjected in one or more subsequent steps. For instance, protecting an amine as a carbamate enables other functional groups to undergo selective reactions with electrophiles whereby the carbamate (protected amino group) is left intact. This involves two additional synthetic steps: the step to form the protected intermediate and a deprotection once the additional selective synthetic steps have been completed. The skilled person is well aware about suitable protecting groups as well as the techniques involved in protecting and deportecting a given compound.

### Specific Embodiments

A. General: ¹H and ¹³C NMR spectra are recorded on a 600 MHz spectrometer operating at 600.2 and 150.9 MHz, respectively. The solvent residual peak is used as a standard in the ¹H and ¹³C measurements in CDCl₃ and CD₃OD (7.26 ppm or 77.16 ppm for CDCl₃ and 3.31 ppm or 49.00 ppm for CD₃OD, respectively) (see Gottlieb, H.E.; Kotlyar, V.; Nudelman, A. NMR Chemical Shifts of Common Laboratory Solvents as Trace Impurities. J. Org. Chem. 1997, 62, 7512). The ⁿ*J_{HH}* couplings are calculated from the proton spectra and all J values are given in Hz. HPCCC = High Performance Counter Current Chromatography.

B. Example of Suzuki-Miyaura coupling: Synthesis of 2-(4-(piperazin-1-yl)phenyl)-1H-benzo[d]imidazol-5-amine (**1a**): 2-Bromo-1H-benzoimidazol-6-amine hydrochloride (100 mg, 0.40 mmol), 4-piperazinylphenyl boronic acid pinacol ester (150 mg, 0.52 mmol, 1.3 eq) and triethylamine (494 mg, 680 µl, 4.88 mmol, 12.2 eq) are dissolved in 1,4-dioxane/H₂O (4 ml/1 ml) and Pd(dppf)Cl₂ (100 mg, 0.14 mmol, 0.34 eq) are added to the reaction mixture and stirred for 48 h at 100°C. The reaction mixture is cooled to room temperature and 15 ml of EtOAc/MeOH (1:1) is added and the reaction mixture filtered through small celite bed. Celite bed is washed with small amount of EtOAc/MeOH (1:1) before 25-30 ml of MeOH/NH₃ (97:3) is ran through the celite bed and MeOH/NH₃ (97:3) is evaporated to dryness *in vacuo.* 2-(4-(piperazin-1-yl)phenyl)-1H-benzo[d]imidazol-5-amine 1a (47 mg, 54%) is obtained as an brown powder. ¹H NMR (CD₃OD): δ 7.89 (d, 2H, ³*J*_{HH} = 8.9), 7.33 (d, 1H, ³*J*_{HH} = 8.5), 7.04 (d, 2H, ³*J*_{HH} = 8.9), 6.93 (d, 1H, ⁴*J*_{HH} = 1.8), 6.73 (dd, 1H, ³*J*_{HH} = 8.5, ⁴*J*_{HH} = 2.0), 3.26-3.22 (m, 4H), 3.00-2.95 (m, 4H). ¹³C NMR (CD₃OD): δ 153.1, 152.3, 144.5, 139.9, 135.0, 128.6 (2C), 122.7, 117.1 (2C), 116.7, 114.4, 100.4, 48.1 (2C), 45.3 (2C)

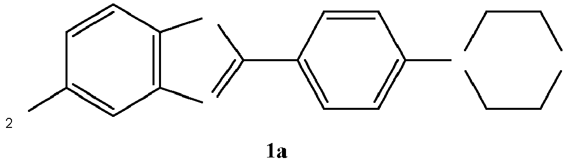

C.1. Example of amidation: Synthesis of N-(2-(4-(dimethylamino)phenyl)-1H-benzo[d]imidazol-5-yl)-2-(2H-indazol-2-yl)acetamide (2a; QHL-24): 2H-lndazol-2-ylacetic acid (28 mg, 0.16 mmol), 2-(4-(dimethylamino)phenyl)-1H-benzimidazol-5-ylamine (40 mg, 0.16 mmol) and DIPEA (24.7 mg, 34 µl 0.19 mmol, 1.2 eq) are dissolved in dry acetonitrile (2 ml) and EDC hydrochloride (37 mg, 0.19 mmol, 1.2 eq) is added to the stirred reaction mixture before HOBt hydrate (29 mg, 0.19 mmol, 1.2 eq) is added and the reaction mixture stirred for 48 h at 40°C. The reaction mixture is evaporated to dryness *in vacuo* and purified by HPCCC using HEMWat solvent system 12. N-(2-(4-(dimethylamino)phenyl)-1H-benzo[d]imidazol-5-yl)-2-(2H-indazol-2-yl)acetamide 2a (19.3 mg, 30%) is obtained as slightly yellow powder. ¹H NMR (CD₃OD): δ 8.10 (s, 1H), 7.95 (d, 1H, ⁴*J*_{HH} = 1.6), 7.87 (d, 2H, ³*J*_{HH} = 9.0), 7.78-7.77 (m, 1H), 7.61.7.58 (m, 1H), 7.47-7.42 (m, 2H), 7.25 (dd, 1H, ³*J*_{HH} = 8.6, ⁴*J*_{HH} = 1.9), 7.21-7.17 (m, 1H), 6.81 (d, 2H, ³*J*_{HH} = 9.0), 5.32 (s, 2H), 3.01 (s, 6H). ¹³C NMR (CD₃OD): δ 166.3, 153.7, 152.0, 140.7, 138.0, 136.7, 133.8, 132.9, 127.5 (2C), 126.8, 124.1, 120.8 (2C), 116.3, 115.5, 114.3, 111.7 (2C), 109.1, 105.4, 51.3, 38.9 (2C).

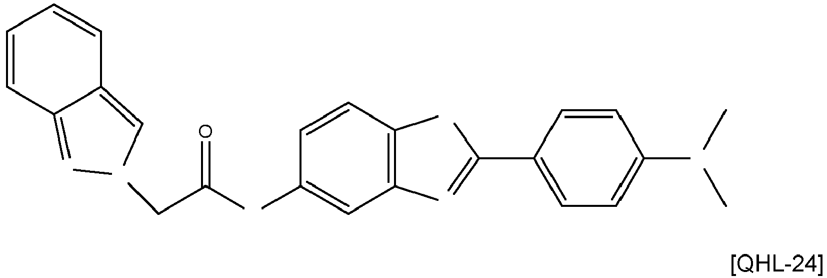

C.2. Example of amidation: Synthesis of N-(2-(4-(dimethylamino)phenyl)-1H-benzo[d]imidazol-5-yl)-2-(1H-indazol-1-yl)acetamide (2b): *1H*-lndazol-2ylacetic acid (28 mg, 0.16 mmol), 2-(4-(dimethylamino)phenyl)-1H-benzimidazol-5-ylamine (40 mg, 0.16 mmol) and DIPEA (24.7 mg, 34 µl 0.19 mmol, 1.2 eq) are dissolved in dry acetonitrile (2 ml) and EDC hydrochloride (37 mg, 0.19 mmol, 1.2 eq) is added to the stirred reaction mixture before HOBt hydrate (29 mg, 0.19 mmol, 1.2 eq) is added and the reaction mixture stirred for 48 h at 40°C. The reaction mixture is evaporated to dryness *in vacuo* and purified by HPCCC using HEMWat solvent system 12. N-(2-(4-(dimethylamino)phenyl)-1H-benzo[d]imidazol-5-yl)-2-(2H-indazol-2-yl)acetamide 2b (16.0 mg, 26%) is obtained as slightly yellow powder. ¹H NMR (CD₃OD): δ 8.33 (s, 1H), 7.98 (d, 1H, ⁴*J*_{HH} = 1.7), 7.89 (d, 2H, ³*J*_{HH} = 9.0), 7.76-7.72 (m, 1H), 7.63.7.60 (m, 1H), 7.47 (d, 1H, ³*J*_{HH} = 8.7), 7.34-7.29 (m, 1H), 7.27 (dd, 1H, ³*J*_{HH} = 8.6, ⁴*J*_{HH} = 1.8), 7.12-7.08 (m, 1H), 6.83 (d, 2H, ³*J*_{HH} = 9.0), 5.37 (s, 2H), 3.03 (s, 6H).

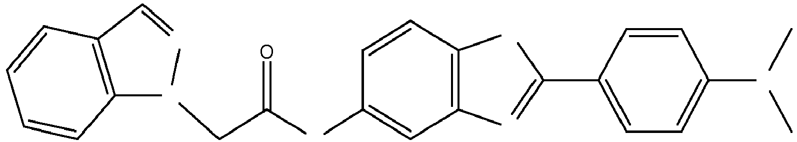

### 5. Target product profiles

### 5.1 Gastrointestinal infections

The compound according to the invention is targeted at virulence mechanisms and OM integrity and is intended to reduce shedding time and promote clearance if administered in acute gastrointestinal infection. Given the protein sequence conservation of SurA in *E. coli, Shigella flexneri*/*sonnei* and also *Salmonella enterica* our SurA inhibitor is likely to act on all of the above-mentioned species. A SurA inhibitor could be used to prevent spreading of enteropathogen infections e.g. in nursing homes or daycare facilities. Several indications for treatment with a SurA inhibitor are conceivable, e.g. (I) administration in infected persons (where antibiotic treatment is not desirable) will reduce suffering from diarrhea, reduce shedding time, promote clearance and thereby reduce the risk of infection of other persons. (II) Contact persons (e.g. family members, nurses, kindergarten teachers) of infected individuals will take the SurA inhibitor as prophylaxis to avoid infection with the enteropathogen.

Patients suffering from bacterial gastroenteritis present with diarrheal symptoms typically sustaining for few days which might come with additional blood admixtures within the stool. However, long shedding time over weeks and months might as well be possible. If there does exist reasonable suspicion for an infection with *Salmonella, E. coli* or *Shigella* each pathogen can be identified by quick tests (e.g. PCR) within 1 day. Otherwise, the identification of the causative agent will take 2-3 days. Given the diverse courses and durations of diarrheal symptoms, treatment with a SurA inhibitor in acute infection will thus only make sense in some cases (e.g. where a causative pathogen was identified). However, if taking into account the long shedding time associated with *E. coli* infection (EHEC; adults: several days - several weeks; children: more than 1 month) or Salmonellosis (adults: ~ 1 month; children below 5 years: ~ 7 weeks; in severe cases: long-term shedding up to 6 months), a prescription of a SurA inhibitor that significantly reduces this time period is highly desirable to avoid spreading or reinfection in all cases.

### 5.2 Recurrent uncomplicated urinary tract infections

Urinary tract infections (UTI) are some of the most frequent bacterial infections, affecting ~ 150 million people per year worldwide. Approximately 1 % of all ambulatory visits in the US account for patients presenting with urinary tract infections. It was estimated that the total costs of UTI sum up to 3.5 billion U.S.-$ if health care costs and time missed from work are included. Uncomplicated UTI typically affects otherwise healthy individuals and causes morbidity especially in women of all ages. UTIs are caused by a number of Gram-negative and less commonly Gram-positive bacteria, but also fungi. The most frequent cause of uncomplicated UTI is uropathogenic *E. coli,* accounting for ~ 75 % of cases. Uncomplicated UTI is often treated by the prescription of antibiotics which may have severe side effects (long-term alteration of vaginal and gut microbiota, candidiasis, development of resistance in bacterial organisms). If an uncomplicated UTI is observed at least 3 times within 12 months or 2 times within 6 months it is termed recurrent UTI. Recurrence may be caused by bacterial reinfection or by bacterial persistence, with reinfection being the more common mechanism. One measure to avoid recurrence is continuous low-dose antibiosis (6-12 months). This regimen is rather effective, but includes common side effects of antibiosis, i.e. vaginal or oral candidiasis, gastrointestinal symptoms due to disturbance of the endogenous microbiota and induces emergence of bacterial resistance. An effective alternative to antibiotic treatment which targets the ability of UPEC to colonize and persist within the urogenital tract thus is desirable. There is reported evidence that the function of SurA significantly influences the virulence of uropathogenic *E. coli* (impaired adhesion and invasion, reduced virulence in cystitis model, failure to establish intracellular bacterial communities causing recurrent UTI). Thus, the compound according to the invention could be used as an alternative to treat women suffering from recurrent UTI as an alternative to continuous low-dose antibiotics in order to (I) prevent reinfection, avoid side effects of antibiosis and avoid emergence of antibiotic resistance and (II) to prevent intracellular bacterial community formation and enhance efficacy of antibiotic treatment in acute infection.

### 5.3 Re-sensitizer of multiresistant Gram negative pathogens in bloodstream infection

The emergence of multiresistant Gram-negatives (such as *E. coli Ec, K. pneumoniae Kp, P. aeruginosa Pa),* resistant against all 4 clinically relevant classes of antibiotics (4MRGN) is a rising problem in global health care. 4MRGN are resistant against cephalosporins (3^{rd} and 4^{th} generation; e.g. Cefotaxim, Ceftazidime), Acylureidopenicillins (e.g. Piperacillin), Fluorochinolones (e.g. Ciprofloxacin) and Carbapenems (e.g. Imipenem, Meropenem). Patients at risk may be colonized asymptomatically for long time. However, when it comes to serious infection caused by 4MRGN, treatment options are very limited. For 4 MRGN Ec and Kp a combination therapy with the last resort antibiotic Colistin (Polymyxin E with severe side effects such as nephrotoxicity and neurotoxicity) and Tigecyclin is used. Alarmingly, the occurrence and spread of plasmid-mediated Colistin resistance has been reported and it is only a matter of time until when resistances against last resort antibiotics will spread and render them also ineffective. Alternative and additional treatment options would thus be highly desirable. The inventors claim that a SurA inhibitor like the compound according to the invention could be used to (re)sensitize 4MRGN to treatment with e.g. Cefepime or Ceftazidime to facilitate treatment with standard antibiotics and in the case of Ec, Pa and Kp infection avoid treatment with nephrotoxic Colistin in seriously ill patients. At the same time a SurA inhibitor will promote clearance of infection by the serum complement system.

## Claims

1. A compound of the following formula 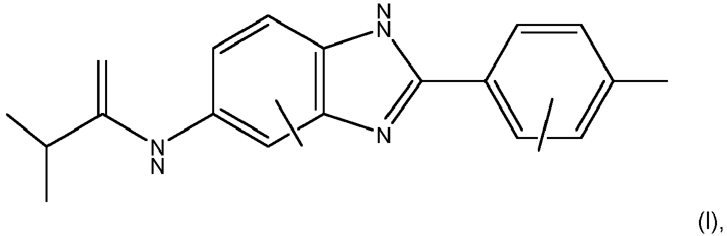 wherein
R₁ and R₄ are independently selected from the group consisting of alkyl, aryl, heteroaryl, cycloalkyl with 3-12 C-atoms, and heterocycloalkyl with 3-12 C-atoms;
R₂ is selected from the group consisting of alkyl, aryl, hetero aryl; and
R₃ is independent from each other a functional group having a molecular weight of 200 g/mol or less,
and the salts thereof, the solvates thereof and the solvates of the salts thereof.

2. Method for preparing a compound of the following formula 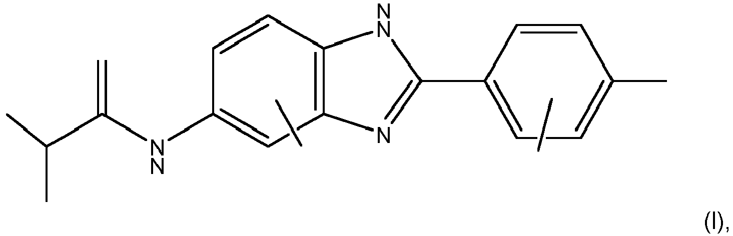 comprising:
- reacting a compound of the following formula 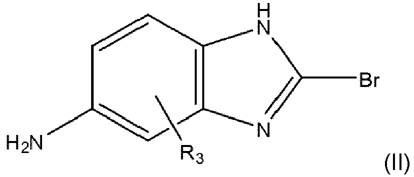 with a compound of the following formula 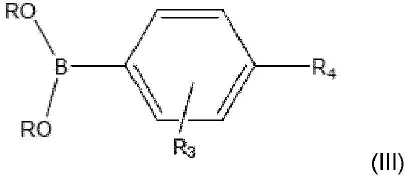
to obtain a compound of the following formula 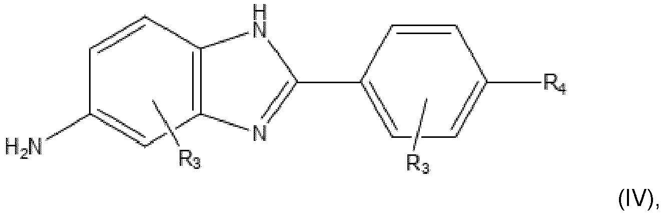 and
- reacting the compound of the formula (IV) with (CHR₁R₂)COR" to obtain the compound of the formula (I), wherein
R₁ and R₄ are independently selected from the group consisting of alkyl, aryl, heteroaryl, cycloalkyl with 3-12 C-atoms, and heterocycloalkyl with 3-12 C-atoms;
R₂ is selected from the group consisting of alkyl, aryl, and heteroaryl; and
R₃ is independent from each other a functional group having a molecular weight of 200 g/mol or less, and
R" is selected from the group consisting of OH, OOH, Cl, Br, OR, OOCOR, wherein
R" is selected from the group consisting of alkyl with 1-8 C-atoms, cycloalkyl with 3-8 C-atoms, and aryl.

3. Compound of claim 1 or method of claim 2, wherein at least one of R₁, R₂, R₃, and R₄ is as follows:
R₁ is selected from the group consisting of an indene having 1-3 C substituted with N, a naphthalene having 1-3 C substituted with N, and dicyclopentadiene having 1-3 C substituted with N;
R₂ is selected from the group consisting of H, OR, SR, NHR, NRR', wherein R and R' are independently selected from the group consisting of alkyl with 1-8 C-atoms, cycloalkyl with 3-8 C-atoms, and aryl;
R₃ is selected from the group consisting of OH, NH₂, SH, COOH, F, Cl, Br, and I; and
R₄ is selected from the group consisting of NH₂, NHR, NRR', and C3 -C12 cyclo alkyl, wherein R and R' are independently selected from the group consisting of alkyl with 1-8 C-atoms, cycloalkyl with 3-8 C-atoms, and aryl.

4. The compound of any of claims 1 and 3 or method of any of claims 2 and 3, wherein the compound of the formula (I) is N-(2-(4-(dimethylamino)phenyl)-1H-benzo[d]imidazol-5-yl)-2-(1H-indazol-1-yl)acetamide, preferably having the following formula

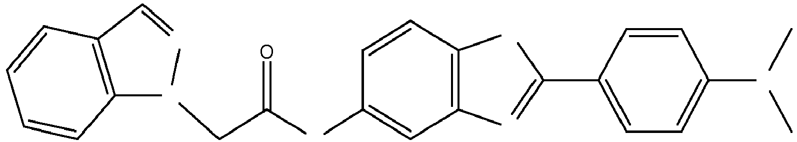

5. The compound of any of claims 1 and 3 or method of any of claims 2 and 3, wherein the compound of the formula (I) is N-(2-(4-(dimethylamino)phenyl)-1H-benzo[d]imidazol-5-yl)-2-(2H-indazol-2-yl)acetamide, preferably having the following formula

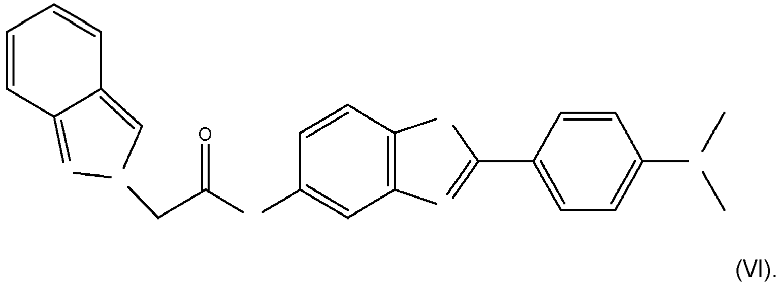

6. The compound of any of claims 1, 3 - 5 for use in the treatment and/or prophylaxis of a disease, preferably an infectious disease, further preferably a bacterial disease, further preferably an infection by a Gram-negative bacterium, highly preferably a bacterium selected from the group consisting of: *Escherichia coli, Pseudomonas ssp, Klebsiella spp., Yersinia spp., Shigella spp., Serratia spp., Morganella spp., Enterobacter spp.*

7. The compound for use of claim 6, wherein said infectious disease is caused by a multi-drug resistant (MDR) bacterium, preferably MDR Gram negative bacterium, preferably an ESKAPE bacterium, including *Klebsiella pneumoniae, Acinetobacter baumannii, Pseudomonas aeruginosa,* and *Enterobacter spp.*

8. The compound for use of claim 6 or 7, wherein said infectious disease is caused by a SurA-expressing bacterium.

9. The compound of any of claims 1 and 3 - 8 for use in the treatment and/or prophylaxis of urinary tract infections (UTI).

10. The compound of any of claims 1 and 3 - 8 for use in the treatment and/or prophylaxis of gastrointestinal infections.

11. The compound of any of claims 1 and 3 - 10 for use in the treatment and/or prophylaxis of blood stream infections, preferably to re-sensitize an MDR bacterium for antibiotics.

12. The compound of any of claims 1 and 3 - 11 for use as a SurA inhibitor.

13. A pharmaceutical composition comprising compound of any of claims 1 and 3 - 12 and a pharmaceutically acceptable carrier.

14. The pharmaceutical composition of claim 13 additionally comprising an antibiotic compound.

15. A method for the treatment and/or prophylaxis of a disease, preferably an infectious disease, of a living being, comprising the administration of the compound of any of claims 1 and 4 - 12 and/or the pharmaceutical composition of claim 13 or 14, to a living being in need thereof.
